# EUROPEAN PATENT APPLICATION

(11) **EP 1 484 393 A1**
(43) Date of publication of application: **08.12.2004**
(21) Application number: 03703353.7
(22) Date of filing: 21.02.2003
(51) Int. Cl.: C12N 15/00, C12N 5/10, C12Q 1/68

(54) **POLYNUCLEOTIDE FOR TARGET GENE**

(30) Priority: 22.02.2002 JP 2002046889
(71) Applicant: OTSUKA PHARMACEUTICAL CO., LTD., Chiyoda-ku Tokyo 101-8535 (JP)
(72) Inventor: SUZUKI, Mikio, Tokushima-shi, Tokushima 771-0125 (JP); MOMOTA, Hiroshi, Itano-gun, Tokushima 771-0219 (JP); WATANABE, Takeshi, Tokushima-shi, Tokushima 770-0941 (JP)
(74) Representative: HOFFMANN - EITLE
(86) International application number: PCT/JP2003/001913
(87) International publication number: WO 2003/070932

(57) **Abstract**

The present invention provides a single strand polynucleotide sequence comprising a target gene, a complementary strand nucleic acid sequence, and a component sequence.

## Description

### TECHNICAL FIELD

The present invention relates to a single strand polynucleotide sequence having an RNA function suppression activity specific to a target gene, and relates to a method for suppressing the function of the target gene using the polynucleotide sequence.

### BACKGROUND ART

RNA interference is a method based on a phenomenon in which the transcription product (RNA) of a target gene is destroyed by introducing into the cell double stranded RNA having the gene to be targeted ("target gene" hereinafter) and a homologous sequence, that is, double stranded RNA comprising two complementary RNA: one RNA strand having a sense sequence to the target gene, and another RNA strand having an antisense sequence to the target gene (Fire, A., et al., Nature, 391, 806-811 (1998). In this Specification, "double stranded" will be used when two separate and complementary molecules are annealed, and "annealed strand" will be used without distinguishing whether a complementary part is annealed within a single molecule or whether two separate and complementary molecules are annealed. It has been demonstrated that the specificity and gene suppression effect of the interference method is higher than that of the antisense method, and the effective concentration is lower. It is known that the annealed strand structure in invertebrates is not specifically restricted in length, that the longer the annealed strand part, the higher the efficiency, and that the gene specific RNA interference phenomenon is produced even when using annealed strand RNA exceeding 30 base pairs. On the other hand, it is known that in vertebrates introduction of long chain double stranded RNA having annealed strand parts that exceed 30 base pairs activates the interferon system, and non-specific suppression of gene function of a broad range of genes other than the target gene (non-specific decomposition of RNA, non-specific suppression of mRNA translation), and cell death, etc. occur. The suppression of gene function utilizing the phenomenon of RNA interference achieves nothing more than a limited effect.

It has been demonstrated that by cutting long sequence annealed strand RNA into small interference RNA (called "siRNA" hereinafter), which have double stranded RNA comprising small-scale RNA with lengths of approximately 21 nucleotides, the small interference RNA will function as molecules with activities to suppress target genes in the cell. T. Tuschl, et al have proven that an RNA interference effect can be produced without causing activation of the interferon system by introducing siRNA having complementarity with the target gene into mammal cells (Elbashir, S.M., et al., Nature, 411, 494-498 (2001)). These siRNA differ from and are superior to long strand annealed strand RNA in that the interferon system is not activated, and that only the specified complementary gene can be specifically suppressed by segments with length of approximately 20 nucleotides. This prior art is a discovery of an efficient gene function suppression system in mammals, and broadened the possibilities of pharmaceutical development and genetic drugs.

The present inventors conducted extensive research directed at: the screening of pharmaceutical target genes using RNA gene function suppression through target RNA decomposition by the siRNA; the *in vivo* assessment of pharmaceutical target genes based on the preparation of knock-down mice; and a more efficient application of pharmaceutical composition (gene therapy agents) to genetic disease.

To achieve the object, it is important to have a structure that transcribes siRNA not only from synthetic RNA, but also from DNA plasmid vector. However, it appears to be difficult to cause expression of 2 differing short strand RNA, and then to cause manifestation of an RNA interference effect in targeted cells by annealing these short strand RNA within the cells.

Here as a result of extensive research, the present inventors perfected the invention by discovering when studying the action of the target gene, that the related activity is suppressed by introducing into the cell a single strand polynucleotide instead of a small double stranded RNA, using a single strand polynucleotide comprising a polynucleotide sequence having an optional component between a short strand polynucleotide complementary to the target gene and a short strand polynucleotide complementary to the polynucleotide.

Objects of the present invention are to provide: a single strand polynucleotide that can suppress and control the functional expression of a targeted RNA or protein by selectively decomposing RNA transcribed from the target gene or selectively suppressing that translation; and a method for suppressing and controlling the function of that target gene by using the single strand polynucleotide. According to the present invention, it is possible to provide: a convenient method for analyzing the functions of genes; a screening method of functional genes that include pharmaceutical target genes; an in vivo assessment method for pharmaceutical target genes using transgenic animals such as knock-down mice, or recombinant viruses; pharmaceutical compositions (gene therapy agents) for genetic diseases and infectious diseases, and transplant organ production animals using knock-down pigs, etc.

### BRIEF DESCRIPTION OF THE DRAWINGS

Fig. 1 shows the RNA function suppression effect based on the polynucleotides for a target gene of the present invention;
Fig. 2 shows the RNA function suppression effect based on a Lamin A/C gene function suppression vector; and
Fig. 3 shows the RNA function suppression effect of a firefly luciferase gene function suppression vector.

### DISCLOSURE OF THE INVENTION

Specifically, the present invention can provide a polynucleotide sequence for a target gene that is an isolated or purified single strand polynucleotide sequence comprising continuous (I) + (II) + (III) components, and that is a polynucleotide sequence for a target gene that has an activity to suppress RNA function in relation to RNA complementary to either the (I) or (III) components; where, the component (III) is 15 to 30 or 18 to 25 continuous polynucleotide sequences having a complementary sequence to the target gene; component (II) is a nucleotide sequence of a length from 0 base to 10 kilobases (where, 0 base means a bond) or a non-nucleotide sequence; and component (I) is a polynucleotide sequence containing a sequence complementary to component (III), or a polynucleotide sequence for a target gene in which the nucleotide sequence comprising the 15 to 30 or 18 to 25 continuous polynucleotide sequences contains DNA or RNA.

In addition, the present invention can provide a polynucleotide sequence for a target gene wherein the polynucleotide sequence and/or complementary polynucleotides of the component (I) or (III) further have a sequence comprising from 1 to several U, T, G, C, or A bases on at least one terminal, or have deleted, substituted or added inside of the complementary sequence. The target gene may be RNA that codes for protein containing mRNA, tRNA, rRNA, Xist, functional RNA that does not code for protein containing adenovirus VA RNA, and virus genome RNA.

Further, the present invention can provide a polynucleotide sequence for target genes comprising a single strand RNA of the SEQ ID No. 1 or 2, wherein the polynucleotide sequence is a single strand polynucleotide obtained by chemical synthesis or gene recombinant technology.

In addition, the present invention provides: a sequence for a target gene wherein the component (II) of the polynucleotide sequence for a target gene comprising a single strand RNA is either a nucleotide sequence or a non-nucleotide sequence, or is comprised by a combination of these; a sequence for a target gene comprising a nucleotide sequence wherein the nucleotide sequence of component (II) has 1 base or more and less than 10 kilobases and a length of from 1 to several hundred bases (for example, 700 bases, 500 bases or 300 bases); a sequence for a target gene comprising component (II) indicating a nucleotide sequence of a length of from 1 or more to less than several dozen bases (for example, 70 bases, 50 bases or 30 bases); a nucleotide sequence of a length of from 1 to 20 bases; or a sequence for a target gene comprising the component (II) indicated in the SEQ ID No. 3 or 4. In addition, the present invention provides the sequence for a target gene wherein the nucleotide sequence or the non-nucleotide sequence of component (II) is PNA, a cytoplasm translocation sequence, a sequence having a decoy activity, an interferon induction suppressing sequence, a sequence having any of RNase suppression activity, antisense activity, ribozyme activity, or transfer RNA, or a combination of these.

The present invention provides a sequence for a target gene or a recombinant vector and a manufacturing method thereof wherein the sequence for a target gene is inserted in a recombinant vector.

The present invention provides a screening method for pharmaceutical target genes or genes having a useful function using the sequence for a target gene.

Further the present invention provides a pharmaceutical composition or a gene therapy agent comprising a pharmaceutical composition wherein the active ingredient is the sequence for a target gene or a recombinant vector.

In addition, the present invention provides a method for synthesizing nucleotides for target genes comprising the steps of:
(i) creating a single strand nucleotide comprising components (I) and (II) such that several nucleotides of the 3' terminal of component (II) are complementary to several nucleotides of component (I) or (II);
(ii) synthesizing component (III) based on nucleotide synthesis enzyme activity using this single strand nucleotide comprising components (I) and (II), or introducing this single strand nucleotide comprising components (I) and (II) into a cell and synthesizing component (III) based on the nucleotide synthesis enzyme activity present inside the cell.

In addition the present invention provides a nucleotide for a randomized target gene obtained by the previously described method wherein the components (I) and (III) are random oligonucleotides.

The present invention provides a method for suppressing the function of a target gene in cells or tissues by introducing an isolated or purified single strand polynucleotide sequence comprising continuous components (I) + (II) + (III) in the cells or tissues, and suppressing the function of the target gene to have RNA function suppression activity in relation to RNA having a complementary sequence to either of the components (I) or (III) (where, the component (III) is a continuous 15 to 30 or 18 to 25 polynucleotide sequences having a sequence complementary to the target gene; the component (II) is a nucleotide sequence or non-nucleotide sequence with a length of from 1 base to 10 kilobases; and the component (I) is a polynucleotide sequence containing a sequence complementary to component (III) or a substance in which the nucleotide sequence comprising a continuous 15 to 30 or 18 to 25 polynucleotides contains DNA or RNA).

The present invention can provide a method for suppressing the function of a target gene wherein the sequence of either the components (I) or (III) are a polynucleotide sequence for a target gene that has one to several bases of U, T, G, C, or A on at least one terminal, or has deleted, substituted or added internally.

Further, the present invention can provide a method for suppressing the function of a target gene wherein the polynucleotide sequence is a single strand polynucleotide sequence obtained by chemical synthesis or genetic recombination technology, and is a polynucleotide sequence for a target gene comprising the single strand RNA of SEQ ID No. 1 or 2.

Moreover, the present invention provides a method using: a sequence for a target gene wherein the component (II) of the polynucleotide sequence for a target gene comprising a single strand RNA used in the method for suppressing the function of a target gene is either a nucleotide sequence or a non-nucleotide sequence, or is comprised by a combination of these; a sequence for a target gene comprising a nucleotide sequence wherein the nucleotide sequence of the component (II) has 1 base or more and less than 10 kilobases and a length of from 1 to several hundred bases (for example, 700 bases, 500 bases or 300 bases); a sequence for a target gene comprising the component (II) indicating a nucleotide sequence of a length of from 1 or more to less than several dozen bases (for example, 70 bases, 50 bases or 30 bases); a nucleotide sequence of a length of from 1 to 20 bases; or a sequence for a target gene comprising the component (II) indicated in the SEQ ID No. 3 or 4.

In addition, the present invention provides a method for suppressing the function of a target gene using the sequence for a target gene wherein the nucleotide sequence or the non-nucleotide sequence of component (II) is PNA, a cytoplasm translocation sequence, a sequence having a decoy activity, an interferon induction suppressing sequence, a sequence having any of RNase suppression activity, antisense activity, ribozyme activity, or transfer RNA, or a combination of these. The target gene may be RNA that codes for protein containing mRNA, tRNA, rRNA, Xist, functional RNA that does not code for protein containing adenovirus VA RNA, and virus genome RNA.

The present invention provides a method for suppressing the function of a target gene wherein the nucleotide sequence comprising a continuous 15 to 30 or 18 to 25 polynucleotides of the aforementioned component (III) contains DNA or RNA; or a method for suppressing the function of a target gene wherein the sequence of either the components (I) or (III) has one to several bases of U, T, G, C, or A on at least either terminal, or has deleted, substituted or added internally; or a method for suppressing the function of a target gene wherein the polynucleotide sequence is obtained by chemical synthesis or genetic recombination technology.

In addition, the present invention provides a method for suppressing the function of a target gene wherein the single strand RNA of SEQ ID No. 1 or 2 is used.

Moreover, in the method for suppressing the function of a target gene, the present invention provides a method wherein the component (II) of the sequence for a target gene is either a nucleotide sequence or a non-nucleotide sequence, or is comprised by a combination of these; and the component (II) comprises a nucleotide sequence of 1 base or more and less than 10 kilobases, a sequence for a target gene comprising a nucleotide sequence of a length of from 1 to several hundred bases, a sequence for a target gene comprising a nucleotide sequence of a length of from 1 to several dozen bases, a nucleotide sequence of a length of from 1 to 20 bases or a sequence for a the sequence for a target gene or target gene comprising the component (II) indicated in SEQ ID No. 3 or 4.

In addition, the present invention provides a method for suppressing the function of a target gene using the sequence for a target gene wherein the nucleotide sequence or the non-nucleotide sequence of component (II) is PNA, a cytoplasm translocation sequence, a sequence having a decoy activity, an interferon induction suppressing sequence, a sequence having RNase suppression activity, a sequence having antisense activity, a sequence having ribozyme activity, a sequence having transfer RNA activity, or a combination of these.

Further, the present invention can provide a cultured knockdown cell or tissue or non-human animal or plant produced: by specifically decomposing or selectively suppressing the translation of RNA transcribed from the target gene or RNA that is the target gene; by a method for suppressing the function expression of targeted RNA or protein, or a method for suppressing expression of the target gene by a method for specifically decomposing the target gene; or by any of the methods described above. In addition, the present invention can provide a method for testing the function of a target gene in cells or tissues or non-human animals or plants by introducing an isolated or purified single strand polynucleotide sequence comprising continuous components (I) + (II) + (III) in the cells or tissues or non-human animals or plants to have RNA function suppression activity in relation to RNA having a complementary sequence to either of the components (I) or (III); as well as a method to detect candidate compounds promoting the RNA function suppression activity in relation to RNA complementary to either of the components (I) or (III) and promoting the functional impairment of the target gene compared to the control comprising, after culturing the cells or tissues together with the test compound, introducing into said cells or said tissues an isolated or purified single strand polynucleotide sequence comprising continuous components (I) + (II) + (III) (wherein the component (III) is a continuous 15 to 30 or 18 to 25 polynucleotide sequence having a sequence complementary to the target gene; the component (II) is a nucleotide sequence or non-nucleotide sequence with a length of from 1 base to 10 kilobases; and the component (I) is a polynucleotide sequence containing a sequence complementary to component (III) or a polynucleotide sequence for the target gene in which the nucleotide sequence comprising a continuous 15 to 30 or 18 to 25 polynucleotides contains DNA or RNA).

In addition, the present invention can provide a method for screening substances that functionally interact with the target gene in cells or tissues or non-human animals or plants by introducing an isolated or purified single strand polynucleotide sequence comprising continuous components (I) + (II) + (III) in the cells or tissues or non-human animals or plants to have RNA function suppression activity in relation to RNA complementary to either of the components (I) or (III) (wherein, the component (III) is a continuous 15 to 30 or 18 to 25 polynucleotide sequence having a sequence complementary to the target gene; the component (II) is a nucleotide sequence or non-nucleotide sequence with a length of from 1 base to 10 kilobases; and the component (I) is a polynucleotide sequence containing a sequence complementary to component (III) or a polynucleotide sequence for the target gene in which the nucleotide sequence comprising a continuous 15 to 30 or 18 to 25 polynucleotides contains DNA or RNA).

The present invention can provide a screening method for identifying compounds to stimulate or suppress functions related to a target gene in cells or tissues or non-human animals or plants by introducing an isolated or purified single strand polynucleotide sequence comprising continuous components (I) + (II) + (III) in the cells or tissues or non-human animals or plants to have RNA function suppression activity in relation to RNA complementary to either of the components (I) or (III), selected from the group of methods of:
(a) using labeling directly or indirectly bound to a candidate compound to measure the binding of the candidate compound and a polypeptide of an amino acid sequence that is coded by the target gene, or a target gene expression product (or a cell or membrane thereof that carries the polypeptide of an amino acid sequence that is coded by the target gene, or a target gene expression product), or a fusion protein thereof;
(b) measuring in the presence of a labeled competition substance the binding of a candidate compound and a cell into which the single strand polypeptide sequence has been introduced (or cells or the membrane thereof carrying the single strand polypeptide sequence), or a fusion substance thereof;
(c) using a detection system applied to a cell or cell membrane carrying a polypeptide of an amino acid sequence that is coded by the target gene or an expression product of the target gene to determine whether or not a candidate compound has a signal produced by suppressing or activating the polypeptide or expression product of the target gene based on the single strand polynucleotide sequence;
(d) preparing a mixture by simultaneously mixing a candidate substance and a solution containing an amino acid sequence that is coded by the target gene or an expression product of the target gene, measuring the activity of the polypeptide or the expression product of the target gene in the mixture, and comparing the activity of the mixture with that of a standard; and
(e) detecting the effect in the cell that the candidate compound has on the mRNA that codes the polypeptide of the amino acid sequence that is coded by the target gene and on the product of the polypeptide of the amino acid sequence coded by the target gene (where, the component (III) is a continuous 15 to 30 or 18 to 25 polynucleotide sequence having a sequence complementary to the target gene; the component (II) is a nucleotide sequence or non-nucleotide sequence with a length of from 1 base to 10 kilobases; and the component (I) is a polynucleotide sequence containing a sequence complementary to component (III) or a polynucleotide sequence for the target gene in which the nucleotide sequence comprising a continuous 15 to 30 or 18 to 25 polynucleotide contains DNA or RNA).

The indication by numbers for the amino acids, peptides, base sequences, and nucleic acids in this Specification conform to IUPAC-IUB regulations [IUPAC-IUB Communication on Biological Nomenclature, Eur. J. Biochem., 138: 9 (1984)], the "Guidelines for Preparing Specifications etc. Containing Base Sequences and Amino Acid Sequences" (ed. Japan Patent Office), and the customary codes in the applicable fields. In addition, the methods of synthesizing DNA, manufacturing vectors (expression vectors) containing exogenous genes, and manufacturing the host cells genetically modified using said vectors and the expression proteins that the host cells secrete can be easily manufactured and obtained using common genetic engineering procedures [Refer to Molecular Cloning 2d Ed, Cold Spring Harbor Lab. Press (1989); Continuing Course in Biochemistry Experiments "Gene Research Methods I, II, III", Japan Biochemistry Society Ed, (1986), etc.).

Substances deduced from polynucleotide sequences for target genes called "uGL3. 12RNA" and "uGL3. 7RNA" indicated in the examples to be described later may be cited as a specific examples of genes of the present invention. The base sequences thereof are as indicated in SEQ ID Nos. 1 and 2.

Regarding the polynucleotide sequences, the 52 polynucleotide sequence indicated in SEQ ID No. 1 is a novel polynucleotide sequence for a target gene, and the component (I) is a sequence of 19 oligonucleotides, the component (II) is a sequence of 12 oligonucleotides, and the component (III) is a sequence of 21 oligonucleotides. The 45 polynucleotide sequence indicated in SEQ ID No. 2 is a novel polynucleotide sequence for a target gene, and the component (I) is a sequence of 19 oligonucleotides, the component (II) is a sequence of 7 oligonucleotides, and the component (III) is a sequence of 21 oligonucleotides.

In the present invention the meaning of "gene" encompasses not only double stranded DNA, but also the single strand DNA of the sense and antisense strands that comprise that double stranded DNA, as well as double stranded and single strand RNA; and there is no limitation as to length. Consequently, unless specifically mentioned, included in the genes of the present invention are double stranded DNA including human genome DNA, single strand DNA (sense strand) including cDNA, single strand DNA (antisense strand) having a sequence complementary to the sense strand, RNA including the virus genome, and any related fragments.

In the present invention, "gene" includes leader sequences, coding regions, exons and introns. RNA, DNA, PNA (peptide nucleic acid), etc. may be cited as examples of polynucleotides. DNA includes cDNA, genome DNA, and synthetic DNA; RNA includes mRNA, tRNA, rRNA, UsnRNA (uridine-rich small nuclear RNA), virus genome, virus mRNA, 5sRNA, transfer RNA, and ribozymes, as well as polyamines that can form complementary bonds with the PNA or natural nucleotides; and polypeptides having a specified amino acid sequence include fragments, homologs, derivatives and variants thereof.

Mutations means polynucleotide sequences that do not substantially change the function of the coded polypeptide: naturally occurring allele mutants, mutants not present in nature, and mutants having one or more deletions, substitutions, additions and insertions.

Further, the alteration of these amino acid sequences (mutations, etc.) may occur in nature, for example, by spontaneous mutation and modification after translation, or may be artificially produced using naturally derived genes (for example, the genes in the concrete examples of the present invention).

The polypeptides include alleles, homologs and natural variants that are at least 90%, preferably 95%, more preferably 98%, and most preferably 99% homologous.

A polynucleotide sequence for a target gene of the present invention is defined as a sequence of single strand comprising a nucleotide or non-nucleotide sequence comprising three components; and said components are defined as a series of single stranded polynucleotide sequences comprising a single strand in the order of component (I) and component (II) and component (III).

The single-stranded polynucleotide sequence comprising the three components is either conjugated after producing the various components independently, or may be produced as a single element. The polynucleotide sequence for a target gene may be chemically synthesized, or produced using genetic recombination technologies. A person skilled in the methods generally used in this field can use these technologies to obtain polynucleotide sequences for target genes that fully meet the objectives, and to obtain isolated or purified single strand polynucleotide sequences. In the above, the synthesis of DNA may be conducted by chemical synthesis using the phosphoramidite method or the triester method, or may be conducted by using commercially available automatic oligonucleotide synthesizer. Moreover, the double stranded fragments necessary in order to insert in an expression vector or to amplify the polynucleotide sequences for a target gene of the present invention may be obtained from single strand products chemically synthesized by synthesizing complementary strands and annealing together with the applicable strands under appropriate conditions, or by adding complementary strands using DNA polymerase together with a suitable primer sequence.

The synthesized RNA or DNA may be purified using PAGE or anion exchange HPLC, etc.

The synthesis of RNA is more difficult than the synthesis of DNA because RNA has a hydroxyl group at 2', and a protecting group at 2' will be necessary, and the yield may be greatly reduced by the desalination and deprotection after production, and that strand stability may be lost. However, by using orthoester (2'-ACE) to protect 2', it is possible to conduct stable RNA synthesis, and in the method, after protecting 2' with the 2'-ACE synthesis RNA oligonucleotide made by Dharmacon Co., desalination and deprotection can be easily conducted by using a volatile buffer at the time of use (http://dharmacon.com/sirna.html). The synthesis of RNA may also be conducted by the phosphoramidite method using the commercially available ABI3900 high throughput DNA synthesizer, etc. manufactured by Applied Biosystems and RNA synthesis reagents.

The intended polynucleotides are preferably chemically synthesized using, for example, a protected ribonucleotide phosphoramidite method, the 2'-ACE method and a suitable deoxyribonucleic acid/RNA synthesizer. The previously described polynucleotide synthesis may be independently synthesized using commercially available deoxyribonucleic acid/RNA synthesizers and following the related user manuals, or, synthesis of this kind of polynucleotide may be easily contracted out to subcontracting companies or departments in this field. The synthesis of deoxyribonucleic acid/RNA may, for example, be subcontracted to Dharmacon Research Co. (Lafayette, CO, USA), Genset Oligos (Genset Oligos Co.: http://www.gensetoligos.com/) Ambion Co., Xeragon (http://www.xeragon.com), Peribio Science Co. (http://perbio.com/) or ChemGenes Co. (http://www.chemgenes.com).

The following methods, or a combination of these methods, may be cited as examples of the method for preparing polynucleotide sequences for a target gene of the present invention: genetic engineering methods such as [Methods in Enzymology, 154, 350, 367-382 (1987) ibid, 100, 468 (1983); Nucleic Acids Res., 12 9441 (1984); Continuing Course in Biochemistry Experiments 1 "Gene Research Methods II ", Japan Biochemistry Society Ed., p105.(1986) etc.], and chemical synthesis means such as the phosphoric acid triester method and the amidite phosphate method [J. Am. Chem. Soc., 89, 4801 (1967); ibid, 91, 3350 (1969); Science, 150, 178 (1968); Tetrahedron Lett., 22, 1859 (1981); ibid, 24, 245 (1983)].

The component (I) or (III) of the polynucleotide sequence for a target gene of the present invention may be partially or completely complementary to the RNA of the targeted gene, and the targeted RNA may target all of the RNA present in cells including mammals such as mRNA, tRNA, rRNA, virus genome, virus mRNA, Xist RNA. The component (III) including parts complementary with the RNA of the target gene may be of a length from 15 to 30 continuous polynucleotide sequences, preferably from 18 to 25 polynucleotide sequences, and most preferably from 19 to 21 polynucleotide sequences. Moreover, for the gene region selected as the target for part or all of the component (III) to complement it is preferable to select exon sites from 50 to 100 bases downstream of the initiation codon that codes for the gene , and it is better to avoid the 5' and 3' UTRs. Moreover, higher selectivity for the sequence of the site complementary to the target gene is preferable. As a region to be selected within the region, the sequence region called AA(N19)TT containing about 50% of G or C may be cited as an example of a region complementary to all or part of the component (III) to be selected. If the previously described kind of sequence cannot be discovered, it is possible to use AA(N21) or CA(N21) as the terminal site.

The component (III) may be either RNA or DNA.

The decision on the region complementary to the target gene for the component (III) of the polynucleotide sequence for a target gene of the present invention may be made by conducting a NCBI blast search. In addition, a polynucleotide sequence to suppress the function of the target gene may be selected and used for the purpose of the present invention by using a component (III) that was synthesized to make an optional sequence.

The component (I) of the polynucleotide sequence for a target gene of the present invention may be a sequence complementary to the sequence of the component (III), or may be complementary to more than 50% of the component (III), and may be complementary to the gene region selected as the target in the same way as the sequence of the component (III). Further, the component (III) of the polynucleotide sequence for a target gene of the present invention may have a length one or several bases longer than the length of the sequence of the component (I), for example, two uracil (U) bases may be added. Moreover, either of the components (I) or (III) of the polynucleotide sequence for a target gene may have at least 1 to several bases of U, T, G, C or A on any terminal, or may have deleted, substituted or added to the interior.

Next, the component (II) of the polynucleotide sequence for a target gene of the present invention may be any nucleotide or non-nucleotide sequence, or a combination thereof. Examples of the nucleotide sequence include a sequence comprising a nucleotide sequence of 1 base or more and less than 10 kilobases, preferably a nucleotide sequence of a base length of from 1 to several hundred bases, a nucleotide sequence of a base length of from 1 to several dozen bases, or a nucleotide sequence of a base length of from 1 to 20 bases, or comprising a component (II) that uses a mechanism provided in the cell, such as splicing, to produce nucleotides in the cytoplasm with a nucleotide sequence of a base length mentioned above, a nucleotide sequence comprising the sequence indicated in SEQ ID No. 3 or 4 as shown in the examples of the present invention described below. The nucleotide sequence may comprise a sequence complementary to that of components (I) or (III). Moreover, PNA (peptide nucleic acid) of a chemical synthesis analog having a polyamide skeleton similar to nucleic acid may be cited as a non-nucleotide sequence of the component (II) of the polynucleotide sequence for a target gene of the present invention. Examples of nucleotide sequences comprising component (II) include cytoplasm translocation sequences such as poly A, tRNA, UsnRNA, and CTE sequences derived from retrovirus, NF kappa β binding sequence, E2F binding sequence, SSRE, sequences having decoy activity such as NF-AT, interferon induction suppression sequences such as adenovirus VA1 or VA2 RNA, sequences having RNase suppression activity, antisense activity, and ribozyme activity, transfer RNA or marker sequences for specifying expression sites, and selection marker sequences in E. coli for detection; or sequences that are combinations thereof. Functional sequences requiring partial annealed strands such as those with decoy activity may be prepared by including complementary nucleotides. Further, within cells that have an intron donor sequence inside of component (II) and a sequence containing an acceptor sequence necessary for splicing, and that have a splicing mechanism based on this, part of component (II) may be cut out and reconnected. Depending on the composition of these components (II), desirable characteristics may be obtained such as effect to enhance the RNA function suppression effect, and stability as a polynucleotide sequence for a target gene or as a substance with a sequence for a target gene.

In addition, the polynucleotide sequence for a target gene of the present invention may even be manufactured using gene recombination technology. For example, after chemically synthesizing a single strand polynucleotide sequence comprising continuous components (I) + (II) + (III) described above, a PCR primer may be produced based on an optional sequence (containing a promoter sequence and a terminator sequence) that can be canned to both ends of the sequence or to the outside thereof, and once the sequence has been made into double stranded DNA, amplification can be conducted using PCR. In the description above, all components may be chemically synthesized as a whole, or the various components may be connected after chemical synthesis. Moreover, the promoter may be suitably arranged in relation to the transcription initiation point using T7 promoter in *in vitro* transcription and E.coli, with CMV promoter in eukaryotic cells, and U6 promoter and H1 promoter in a eukaryotic cell PolIII system. The terminator can cleave the product after transcription at the same site using another transcription terminating sequence or a self-cleaving ribozyme, etc. Or, amplified double stranded DNA may be obtained by using a restriction enzyme recognition site that can add to the terminal thereof, connecting to a vector with the restriction enzyme, amplifying, and producing the desired polynucleotide sequence for a target gene. There is currently no technical difficulty in this field with incorporation in various types of plasmid vectors.

Further, the polynucleotide sequence for a target gene of the present invention may be produced with gene recombination technology using the fact that components (I) and (III) have complementarity. For example, the complementary sequences of the component (I) and the component (III) are produced using chemical synthesis or gene recombination technology so that the several nucleotides of the 3' terminal of the component (II) (partial sequence of the component (I)) are complementary to several nucleotides of the component (III). Enzymes including nucleotide synthesis enzymes containing DNA polymerase or RNA polymerase may be used to synthesize double stranded polynucleotides comprising the components (I), (II), (III) from the nucleotide 3' terminal of the component (I) and the nucleotide 3' terminal of the component (III) that have formed a complementary annealed strand comprising several bases. The polynucleotide sequence for a target gene of the present invention may be synthesized thereby.

The component (III) may be synthesized singly using chemical synthesis or gene recombination technology, annealing may be conducted using the complementarity with the component (I), and the 5' terminal of the component (III) and the 3' terminal of the molecule comprising the components (I) and (II) may be connected chemically, or connected using an enzyme such as ligase.

Further, for example, the components (I) and (II) are produced using chemical synthesis or gene recombination technology so that the several nucleotides of the 3' terminal of the component (II) are complementary to several nucleotides of the components (I) or (II). This molecule comprising the components (I) and (II) may be introduced into cells, and the component (III) may be synthesized by the nucleotide synthesis enzyme activity present in the cell. Nucleotide synthesis enzymes may be introduced into the cells by genetic recombination or by viral infection, etc. Specifically, molecules comprising only components (I) and (II) also may be included in the present polynucleotides for a target gene.

The vectors that express the polynucleotides for a target gene comprising these components (I) and (II), or the polynucleotides for a target gene comprising these components (I) and (II) and (III) may be synthesized based on the vector construction described above.

To cultured cells, it is possible to introduce polynucleotide sequences for a target gene or expression vectors of a polynucleotide sequence for a target gene, which have different molecules, and which can suppress the function of the target gene, and thereby the cells in which the desired phenotype change by expression has been induced can be selected. It is then possible to isolate the polynucleotide sequences for a target gene or expression vectors of a polynucleotide sequence for a target gene that have been introduced in the cells , to use an NCBI blast search, etc. to search the polynucleotide sequences for a target gene or expression vectors of the gene sequences of a polynucleotide sequence for a target gene for gene sequences that have complementarity to component (I) or (III), and to assay the target genes in which the desired phenotype change by expression has been induced. In order to achieve this objective, it is possible to produce so-called randomized polynucleotides for a target gene by using the complementary synthesis method, complementary ligation synthesis method or the intracellular complementary strand synthesis method. Specifically, component (I) is synthesized using a synthesis form in which any of the nucleotides of A, T, G, C, or U are randomly introduced into various nucleotide sites equivalent to the component (I), and the complementary component (III) is produced by the above methods. Randomized gene target vectors can be produced thereby, and then the target genes in which the desired phenotype change by expression has been induced can be assayed.

Thus, it is possible to easily produce a polynucleotide sequence for a target gene of the present invention as well as a recombinant vector into which said polynucleotide sequence for a target gene is incorporated.

The polynucleotide sequences for a target gene as well as recombinant vectors in which a polynucleotide sequence for a target gene of the present invention is incorporated may be produced by following the conventional methods of gene recombination technology described above based on the sequence information of a sequence for a target gene or a polynucleotide sequence for a target gene provided by the present invention (for example, refer to Science, 224, 1431 (1984); Biochem. Biophys. Res. Comm., 130, 692 (1985); Proc. Natl. Acad. Sci., USA, 80, 5990 (1983), etc.). In addition, a person skilled in the art could easily obtain and purify sequences for a target gene or polynucleotide sequences for a target gene from recombinant expression vectors manufactured by the above.

Moreover, if a vector, in which a sequence for a target gene or a polynucleotide sequence for a target gene of the present invention has been inserted, is divided in two, it is necessary for oligo-RNAs transcribed from separate promoters to meet and associate by chance within a cell that has an extremely large space (a space of 10¹³ fold of the size of the oligo-RNA), and it may be assumed that the function of the type that is divided into two is weak because the efficiency is extremely low. However, the sequence of a component (III), which contains a sequence complementary to the target gene sequence such as a sequence for a target gene or a polynucleotide sequence for a target gene of the present invention, and the sequence of a component (I), which is a sequence complementary to that of the component (III), are made into a single strand, and therefore the production of the vectors of the present invention are extremely significant considering that the sequences are inserted in the cell as a molecule having complementarity and are always in close proximity, and allow annealed strands that destroy RNA to be formed extremely efficiently.

Normally, exposed terminals are prone to attack by nuclease, and therefore being made into a single strand provides more protection than double stranded as a result of one-sided terminal exposure (the 3' terminal side of the component (I) of the present invention), and increases the stability of the vectors in which a sequence for a target gene or a polynucleotide sequence of a target gene of the present invention has been introduced. Nonetheless, a single strand in which the annealed strand parts exceed 30 base pairs cannot induce suppression of specific gene functions in vertebrates, and have low utility as mentioned above regarding advantages.

Next, the present invention provides a method for suppressing functions of a target gene in cells, tissues, non-human animals and plants using a sequence for a target gene or a polynucleotide sequence of a target gene of the present invention. One concrete example of the method includes: as a result of using the polynucleotide for a target gene indicated in SEQ ID Nos. 1 and 2 of the present invention as indicated in the examples to be described later, and administering into HeLa cells the polynucleotide sequence for a target gene targeting the luciferase gene, it was possible to manifest an effect to suppress the luciferase activity of the luciferase gene, the target gene, based on the effect to suppress the RNA function of the RNA of the gene.

More concretely, by conducting an NCBI blast search based on the gene sequence information of the targeted gene, the AA(N19)TT sequence region, which is 50 to 100 bases downstream from the initiation codon of the coding region of the target gene, and which is comprised of about 50% G or C, is selected as the region complementary to part or all of the component (I). Preferably, it is better that the region is the region having the targeted gene specific sequence. If the previously described sequence cannot be discovered, the component (III) region is set up to be complementary by taking AA(N21) or CA to be the terminal site, for example, the base of the component (III) is determined to be a 21-oligonucleotide sequence wherein two uracil bases are added to the 3' terminal of the 19-oligonucleotide sequence, and then another base is determined taking the sequence complementary to the 19-oligonucleotide sequence region of the component (III) as the sequence comprising the component (I). Then, an oligonucleotide sequence comprising the RNA sequence of an optional 7 or 12 bases is determined to be the component (II), these are combined, and after using a commercial automatic synthesizer to chemically synthesize, desalinate and purify single strand polynucleotide sequence comprising continuous components (I) + (II) + (III), the RNA for RNA transfection is dissolved in distilled water, a mixed solution of buffer solution (100 mM potassium acetate, 30 mM HEPES-KOH adjusted to pH 7.4, 2 mM magnesium acetate) is prepared, and solutions of various dilution percentages are prepared using PBS and the buffer solutions.

Subsequently, a gene expression vector prepared to have the target gene is produced, the various RNA prepared as above are added, and for example, a reaction solution is produced wherein expression vector of a target gene and synthesized polynucleotides for a target gene of the present invention are added to 50 µL of OPTI-MEM serum-free medium, and when using transfection reagents of a polycation lipid liposome system such as lipofectamine 2000, the introduction efficiency increases. After adding the previously described reagents, for example, after making the HeLa3 cells of an established animal cell line into a confluent state, after pre-culturing using DMEM/10 medium (DMEM/10: D-MEM medium to which 10% bovine fetal serum has been added) under 5% CO₂ at 37° C, the cells are rinsed with PBS, and 0.5 mL of Hela3 cells is added to each well of a 24 well plate. After culturing for 24 hours under 5% CO₂ at 37° C and adding to each well the transfection complex produced as described above, this is cultured a further 24 hours under 5% CO₂ at 37° C. Assuming, for example, that the targeted genes are luciferase genes, the dual-luciferase reporter assay system: #E1910 (manufactured by Promega) is used, and the chemiluminescence of the luciferase activity is measured by luminometer, etc. The measured values of emitted light of the substance to which oligonucleotide sequences for a target gene of the present invention were added were compared using substance to which no sequences were added, or substance to which non-specific oligonucleotides sequences were added. From the results of comparing with the control and taking values such as 50%, 70%, 80%, 90%, 95%, 99%, or 100% suppression as the standard, it is possible to study the effect of gene function suppression as well as the specific RNA function suppression in relation to the target gene of the sequence for a target gene or a polynucleotide sequence for a target gene of the present invention in relation to the RNA of the target gene. It is possible to provide a method for suppressing the function of a target gene by using the methods to introduce in the cells or tissues of the present invention an isolated or purified single strand polynucleotide sequence having continuous (I) + (II) + (III) components, and based on an activity to suppress RNA function of the RNA of genes complementary to the polynucleotide sequence of the component (I) or (III). Moreover, in implementing the present method, as a result of suppressing the function of the RNA of the targeted gene, a change is observed in the function of the gene within the cells and tissues based on suppressing the expression of the gene or suppressing the expression of the proteins of the amino acid sequence coded by the gene. Consequently, the present invention provides a method for suppressing the expression of the gene or a method for suppressing the expression of the proteins of the amino acid sequence coded by the gene by introducing in cells or tissues an isolated or purified single strand polynucleotide sequence having continuous (I) + (II) + (III) components to have an RNA function suppression activity of the RNA of genes complementary to the polynucleotide sequence of the component (I) or (III). With the previously described method, the oligonucleotide sequence for a target gene of the present invention can further heighten functions, including the RNA function suppression activity and the cytoplasm translocation activity, by adding a variety of non-nucleotides or alterations and modifications of the nucleotide sequence to the component (II) as described above.

Moreover, in materials such as cells, tissues, cell lysate and organs used in the tests mentioned above, the target gene used to find the functional changes of the genes and the effects of RNA function suppression of the genes by the RNA function suppression activity of the RNA of targeted genes using the polynucleotide sequence for a target gene of the present invention may be easily produced and obtained by general genetic engineering procedures based on the sequence information of the concrete example of the disclosed gene, for example [refer to Molecular Cloning 2d Ed, Cold Spring Harbor Lab. Press (1989; Continuing Course in Biochemistry Experiments "Gene Research Methods I, II, III", Japan Biochemistry Society Ed, (1986), etc.). Specifically, a cDNA library is prepared following normal methods from a suitable source in which the target gene is expressed, and the desired clone is selected from the library using a suitable probe and antibody specific to the gene of the present invention [Proc. Natl. Acad. Sci., USA., 78, 6613 (1981); Science, 222, 778 (1983), etc.]

A variety of cells, tissues, cell lysate and cultured cells derived therefrom that express the target gene may be cited as the source of the cDNA above. In addition, conventional methods may be conducted for all such procedures as the isolation of complete RNA from these human body fluids, blood or tissues, the isolation and purification of mRNA, and the acquisition and cloning of cDNA. Moreover, there are commercial cDNA libraries, and a cDNA library such as one of the various cDNA libraries commercialized by Clontech Lab. Inc. may be used in the present invention. Or, established cell lines that have been deposited or commercialized such as mouse fibroblast cell NIH3T3, monkey COS7 cells, HeLa cells and human embryonic kidney 293 cells may be used.

As described above, the sequence for a target gene or the polynucleotide sequence for a target gene of the present invention, or a recombinant vector combining these sequences have an activity to suppress the function of the RNA of the targeted gene, and because a method for suppressing gene function that suppresses gene function by this activity can be provided, it is possible to suppress the function of the gene or protein by, for example, suppressing the expression of the RNA of the gene or suppressing the expression of the protein of the amino acid that is coded by the gene. Therefore, by suppressing the expression of a gene that has a high level of expression related to various diseases, a gene that comes to have an injurious function through gene mutation, or a gene derived from a virus, this sequence for a target gene or the polynucleotide sequence for a target gene of the present invention, or a recombinant vector combining these sequences may provide a pharmaceutical composition and a genetic therapeutic agent comprising the pharmaceutical composition that has as the active ingredient a sequence for a target gene or the polynucleotide sequence for a target gene of the present invention, or a recombinant vector combining these sequences.

The sequence for targeting a gene or the polynucleotide sequence for targeting a gene of the present invention, or a recombinant vector combining these sequences suppresses the function of the target gene in this way, and therefore, pharmaceutical compositions which have the sequence for targeting a gene or the polynucleotide sequence for targeting a gene of the present invention, or a recombinant vector combining these sequences as the active component are effective for the treatment of the targeted diseases, that is, diseases related to genes wherein there is a demonstrated relationship with virus derived genes or genes that have an injurious function based on high gene expression or genetic mutation, for example: breast cancer related to c-erbB, and erb genes; cancer related genes such as hst-1, src, fps, abl, myc, jun, and myb; RNA tumor related viruses such as leukemia virus, breast cancer virus, and sarcoma virus; hepatitis viruses such as HBV, and HCV; and diseases with a demonstrated relationship to the presence of chromosomes such as the NPY chromosome, and obesity related to the ANGPTL3 chromosome.

Further, there is the possibility of suppressing the function of genes that induce a rejection response in cross-species or intra-species organ or cell transplants by using the sequence for a target gene or the polynucleotide sequence for targeting a gene of the present invention, or a recombinant vector combining these sequences, and these sequences may also be used to treat neural disease and various types of organ diseases including stem cell transplants.

Consequently, the present invention provides pharmaceuticals that take as the active ingredient pharmaceutical compositions of the present invention, or vectors for treating genes containing the related pharmaceutical compositions, and cells into which the related vectors introduce the sequence for a target gene or the polynucleotide sequence for targeting a gene of the present invention, or a recombinant vector combining these sequences.

Specifically, the present invention provides: sequences for a target gene or polynucleotide sequences for targeting a gene of the present invention; pharmaceutical compositions or vectors to be introduced for gene therapy that contain recombinant vectors that incorporate these sequences; cells into which the vectors are used to introduce the sequence for a target gene or the polynucleotide sequence for a target gene of the present invention, or a recombinant vector incorporating these sequences; and gene therapeutic agents that have as the active component the vectors to be introduced for gene therapy or cells into which the vectors are used to introduce the sequence for a target gene or the polynucleotide sequence for a target gene of the present invention, or a recombinant vector incorporating these sequences.

Further the present invention provides pharmaceuticals that contain as the active ingredient virus vectors to be introduced for gene therapy that contains the sequence for a target gene or the polynucleotide sequence for targeting a gene of the present invention, or a recombinant vector incorporating these sequences; and the pharmaceuticals used in treatment for suppressing the activity caused by high expression of disease related genes, in particular, as an anti-cancer agent for breast cancer related to c-erbB, and erb genes; cancer related genes such as hst-1, src, fps, abl, myc, jun, and myb; and RNA tumor related viruses such as leukemia virus, breast cancer virus, and sarcoma virus; as an anti-hepatitis therapeutic agent for hepatitis viruses such as HBV, and HCV; and as an anti-obesity agent for obesity related to the NPY gene, the ANGPTL3 gene, MGAT gene, and the DGAT gene.

The gene therapy will be described in detail below. The implementation of the gene therapy below is not particularly limited, and customary methods may be used such as chemical, molecular biological, microbiological, recombinant DNA, genetic and immunologic methods. These are cited, for example, in Maniatis (Maniatis, T., et al., Molecular cloning: A laboratory manual (Cold Spring Harbor Laboratory, Cold Spring Harbor, New York (1982)), Sambrook (Sambrook, J., et al., Molecular cloning: A laboratory manual, 2nd Ed. (Cold Spring Harbor Laboratory, Cold Spring Harbor, New York (1981)), Ausbel (Ausbel, F.M., et al., Current Protocols in Molecular Biology, John Wiley and Sons, New York, New York, (1992)), Glover (Glover, D., DNA Cloning, I and II(Oxford Press) (1985)), Anand (Anand, Techniques for the Analysis of Complex Genomes, (Academic Press (1992)), Guthrie (Guthrie, G., et al., Guide to Yeast Genetics and Molecular Biology, (Academic Press) (1991)), and Fink (Fink, et al., Hum. Gene Ther., 3, 11-19 (1992).

In gene therapy or transplant therapy through suppression of gene function, the present invention provides nucleotides having a complementary sequence to the RNA in cells that express disease related genes as described above, and provides a gene therapy method that suppresses or deactivates the activity that induces or promotes the disease states described above by providing a gene function suppression drug for inhibiting translation and RNA function and for suppressing the expression of the disease related gene by suppressing the function of the RNA. The therapeutic method is a method that inhibits the process of transcription or translation, and suppresses the expression of the targeted gene by the sequence for a target gene or the polynucleotide sequence for a target gene of the present invention, or a recombinant vector incorporating these sequences suppressing the original RNA function of cells having the disease related genes. The present method is able to provide a method that uses the specificity of nucleotide complementarity to suppress only the function of abnormal RNA, and is a method that suppresses allele specific function without suppressing normal function. For this purpose a single strand polynucleotide comprising component (III) of the present invention complementary to the RNA of the gene is produced, and can be incorporated as a method for supplying the single strand nucleotide to the target cell.

If the activity to suppress the functional expression of the disease related gene is supplied, it is possible to suppress the activity of the gene function in the receptor cell/target cell. The related activity can be introduced into the targeted cell, and can be maintained outside the chromosome using the sequence for a target gene or the polynucleotide sequence for targeting the gene, or a recombinant vector or plasmid incorporating these sequences, or can be maintained by incorporated within the chromosome using a retrovirus vector.

In gene therapy for cancer using the sequence for a target gene or the polynucleotide sequence for targeting a gene, or a recombinant vector incorporating these sequences, it is possible to obtain the desired anti-cancer effect by incorporating the polynucleotide sequence for targeting a gene or sequence for a target gene into a retrovirus, adenovirus or AAV derived vector, and suppress the function of RNA of the targeted gene by infecting the cells that express the functional activity of the target gene with this vector.

The vectors for introducing the desired gene for both the related incorporation and maintenance outside the chromosome are already well known in the field, and any related well-known vector can be used with the present invention. Examples may include virus vectors or plasmid vectors contain copies of polynucleotide sequences targeting a gene or of a sequence for a target gene related to an expression controlling element, and that can express the applicable polynucleotide sequence for a gene target or sequence for a target gene in the targeted cell. It is also possible to use the previously described common expression vectors as the vector, but preferably vectors prepared using the vectors disclosed in the specification of US Patent No. 5252479 and the specification of PCT International Publication No. WO93/07282 (pWP-7A, pwP-19, pWU-1, pWP-8A, pWP-21, and/or pRSVL, etc.) and pRC/CMV (manufactured by Invitrogen) may be cited as examples of the source vector. Most preferable are the various types of virus vectors described later.

Substances characteristic to the various kinds of disease tissue targeted for treatment of the disease may be suitably applied as the promoter used in vectors utilized in therapy to introduce genes.

Albumin, α-fetoprotein, α1-antitrypsin, transferrin, and trans-styrene, etc. may be cited as concrete examples for the liver. Carbonic anhydrase I, carcinoembryonic antigen, etc. may be cited as examples for the colon. Estrogen, aromatase cytochrome P450, cholesterol side chain incision P450, 17 alpha-hydroxylase P450, etc. may be cited for the uterus and placenta.

Prostate antigen, gp91-fox gene, prostate specific kallikrein, etc. may be cited for the prostate. Erb-B2, erb-B3, β-casein, β-lactoglobin, and whey protein, etc. may be cited for the breast. Active protein C uro-globulin, etc. may be cited for the lungs. K-14-keratin, human keratin 1 or 6, and leucrin, etc. may be cited for the skin.

Neural collagen acid protein, mature astrocyte specific protein, myelin, tyrosine hydroxylase pancreatic virin, glucagon, and Langerhans islet amyloid polypeptide, etc. may be cited for the brain. Thyroglobulin, and calcitonin, etc. may be cited for the thyroid. α1 collagen, osteocalcin, and bone sialo-glycoprotein, etc. may be cited for the bones. Renin, liver/bone/kidney alkaline phosphatase, and erythropoietin, etc. may be cited for the kidneys, and amylase, and PAP1, etc. may be sited for the pancreas.

Further, Pol III promoters such as U6snRNA, 7SK, tRNA, which are known to express short RNA that do not code proteins, may be used as the promoter used in the vector utilized in gene introduction therapy. In addition, artificially altered pol III promoters may be used.

To produce a vector for introducing a sequence for a target gene or a polynucleotide sequence for targeting a gene, the sequence for a target gene or the polynucleotide sequence for targeting a gene may be easily produced and obtained using common genetic engineering methods as previously described based on the base sequence information of the target gene.

The introduction of a vector for introducing the related sequence for a target gene or polynucleotide sequence for targeting a gene into a cell may be implemented following various methods that are already well known in the field related to the introduction of RNA or DNA into cells, especially, for example, electroporation, the calcium phosphate co-precipitation method, virus transformation introduction, and the HVJ envelope method. Genetically transformed cells with a sequence for a target gene or a polynucleotide sequence for targeting a gene may be used per se in the isolated state as a drug for suppressing the activity of a function that is expressed by a gene, or may be used as a model system for therapy research.

In gene therapy, it is possible to introduce the vector for introducing a sequence for a target gene or a polynucleotide sequence for targeting a gene into the target cells of the patient by injecting systemically, or locally into the targeted tissue site of the patients. If administering systemically at this time, the sequence can arrive at any cells that can express the target gene RNA of cancer genes or of tumor viruses at other sites. If the transformed and introduced genes are not incorporated permanently into the chromosomes of the various target cells, this can be achieved by regularly repeating the related administration. Permanent introduction is possible using a retrovirus, etc.

The gene therapy method of the present invention comprises both methods of: the in vivo method that directly administers the materials for introducing the previously described sequence for a target gene or polynucleotide sequence for targeting a gene; and the ex vivo method that removes targeted cells or tissues from the body of the patient, introduces the genes extracorporeally, and then returns the related cells or tissue back inside the body.

It is also possible to introduce directly into the cells the sequence for a target gene, the polynucleotide sequence for targeting a gene, or a sequence wherein the related sequence is amplified by the PCR method, and to cleave the RNA strand.

In addition, the target cells into which the sequence for a target gene, or the polynucleotide sequence for targeting a gene is introduced may be suitably selected by the target of the gene therapy (treatment). For example, other than cancer cells that are known to express cancer genes, specifically, tissues of the breast, kidney, testes and small intestine, cells like lymphocytes, fibroblasts, hepatocytes, hematopoietic stem cells, and adipose cells may be cited as target cells.

The viral and non-viral introduction methods are included in the methods for introducing the sequence for a target gene, or the polynucleotide sequence for targeting a gene in the gene therapy.

The method of using a retroviral vector as the vector may be cited as a viral introduction method. Other viral vectors include adenovirus vector, HIV (human immunodeficiency virus) vector, adeno-associated virus (AAV) vector, herpes virus vector, herpes simplex virus (HSV) vector, and Epstein-Barr virus (EBV) vector.

The following may be used as non-viral gene introduction methods: the calcium phosphate co-precipitation method; the membrane fusion liposome method in which a membrane fusion liposome is prepared by fusing a liposome enclosing a polynucleotide and a deactivated Sendai virus in which the gene is pre-destroyed by ultraviolet light, and then inducing the polynucleotide into the cell by directly fusing with the cell membrane; the method of physically introducing the polynucleotide into the cell by coating plasmids of the nucleotide on gold and using a high voltage discharge; the naked polynucleotide method that directly infuses plasmids of the polynucleotide into the organs or tumors in vivo; the cationic liposome method that introduces into the cells genes embedded in multilamellar positive charge liposomes; and the ligand-DNA complex method, that, in order to introduce the gene only into specific cells and not into other cells, binds the polynucleotide of the present invention with a ligand bonded to a receptor that is expressed inside the targeted cell, and this complex is administered.

As one example, to summarize the production of EBV vector for introducing the sequence for a target gene, or the polynucleotide sequence for targeting a gene of the present invention: the EB virus (Epstein-Barr virus; EBV) is a virus that belongs to the herpes strain isolated from culture cells originating from Burkitt's lymphoma by Epstein, et al in 1964 [Kieff, E. and Liebowitz, D.: Virology, 2nd ed. Raven Press, New York, 1990, pp. 1889-1920]. The EBV has an activity to transform cells, and therefore, in order to make a vector to introduce genes, a virus that lacks this activity to transform cells must be prepared. This deactivation may be implemented as follows.

Specifically, the EBV genome in the proximity of the target DNA that incorporates the desired external gene is cloned. Then, the polynucleotide fragment having the sequence complementary to the external gene and a drug-resistant gene are incorporated, and made into a vector for preparing a recombinant virus. Next, the vector for preparing a recombinant virus that has been cut out by a suitable restriction enzyme is transfected to an EBV positive Akata cell. The recombinant virus produced by homologous recombination can be recovered together with the wild Akata EBV by virus production stimulus based on anti-surface immunoglobulin processing. By infecting EBV negative Akata cell with this and selecting the resistant stock in the presence of the drug, it is possible to obtain Akata cells infected only by the desired recombinant virus without the coexistence of wild EBV. Further, large quantities of the targeted recombinant virus vector may be produced by inducing virus activity in the recombinant virus infected Akata cells.

The production of non-viral vectors that introduce the desired sequence for a target gene, or the polynucleotide sequence for targeting a gene without using a recombinant virus vector may be implemented by using, for example, a gene introduction method based on membrane fusion liposomes. This method directly introduces substance contained in liposomes into cells by activating fusion to the cell membrane by membrane liposome (vesicle comprising a lipid double membrane).

The introduction of antisense oligonucleotides by the membrane fusion liposomes may be conducted, for example, by the method of Nakanishi, et al. [Nakanishi, M., et al., Exp. Cell Res., 159, 399-499 (1985); Nakanishi, M., et al., Gene introduction into animal tissues. In Trends and Future Perspectives in Peptide and Protein Drug Delivery (ed. by Lee, V.H. et al.)., Harwood Academic Publishers Gmbh. Amsterdam, 1995, pp.337-349].

In addition, the antisense polynucleotide introduction methods using cationic liposomes may also be cited as another method for introducing into target cells a sequence for a target gene, or a polynucleotide sequence for targeting a gene using liposomes. This method may be implemented following the method of Yagi, et al. [Yagi, K., et al., B.B.R.C., 196, 1042-1048 (1993)]. This method focuses on the fact that both plasmids and cells have a negative charge, and attempts to heighten the interaction with the cell by giving both the inner and outer surfaces of the liposome membrane a positive charge, and by increasing the uptake of plasmids via static electricity. Here, it is useful to use multilamellar large vesicles (MLV) having a positive charge for the liposomes, but it is possible to introduce the desired sequence for a target gene, or a polynucleotide sequence for targeting a gene by preparing composites with the plasmids using large unilamellar vesicles (LUV) and small unilamellar vesicles (SUV).

The gene therapy of the present invention includes two representative types of methods for introducing the desired genes in the target cells or target tissues.

The first method is a technique (ex vivo) wherein after collecting the target cells from the patient to be treated, the resulting cells are cultured extracorporeally, for example, with the addition of interleukin-2 (IL-2). After the intended sequence for a target gene or polynucleotide sequence for targeting a gene contained in a retrovirus has been introduced, the cells obtained are re-transplanted. The method is suitable for treatment of genetic diseases that are generated by a missing gene, especially ADA deficiency, and also for arterial sclerosis, cancer, and AIDS.

The second method is a technique that directly introduces a gene (direct method) wherein the intended sequence for a target gene or polynucleotide sequence for targeting a gene (polynucleotide containing an oligonucleotide complementary to the RNA of the disease related gene) is infused directly into the body of the patient, and into the target site such as the brain, kidneys, testes, small intestinal tissue.

In more detail, for example, the first method of gene therapy is implemented as follows. Specifically, the mononuclear cells collected from the patient are separated from the monocytes using blood separation equipment; the separated cells are cultured for about 72 hours in a suitable medium such as AIM-V medium in the presence of IL-2; and a vector containing the sequence for a target gene or polynucleotide sequence for targeting a gene to be introduced (polynucleotide containing an oligonucleotide complementary to the RNA of the disease related gene) is added. In order to raise the efficiency of introducing the sequence for a target gene or polynucleotide sequence for targeting a gene, the cultured cells may be centrifugally separated at 2,500 rpm for 1 hour at 32° C in the presence of protamine, and then cultured for 24 hours at 37° C under 10% carbon dioxide gas. After repeating this procedure several times, the cells are cultured for another 48 hours in an AIM-V medium, etc. in the presence of IL-2, the cells are rinsed with physiological saline, and the number of live cells is calculated. The effect of introducing the intended sequence for a target gene or polynucleotide sequence for targeting a gene (polynucleotide containing an oligonucleotide complementary to the RNA of the disease related gene) is confirmed by measuring the efficacy in introducing the sequence for a target gene or polynucleotide sequence for targeting a gene, by measuring the in situ PCR, and, if there is functional activity of the desired target to express the disease related gene, by measuring the extent of that activity.

Moreover, after confirming the safety by conducting safety checks for the presence of bacterial and fungal cultures or mycoplasma contamination in the culture cells, and endotoxin searches, etc., the culture cells in which the sequence for a target gene or polynucleotide sequence for targeting a gene (polynucleotide containing an oligonucleotide complementary to the RNA of the disease related gene) is returned to the patient by drip infusion at a dose expected to be effective. Gene therapy is conducted, for example, by repeating the method at several week to several month intervals.

Here the dosage of the virus vector is suitably selected according to the target cells to be introduced. Normally, it is preferable to adopt a dose, for example, in the range of 1x10³ cfu to 1x10⁸ cfu as viral potency in relation to 1x10⁸ of the target cells.

As another version of the first method above, it is possible to culture, for example, patient cells together with virus production cells containing retrovirus vector containing the intended sequence for a target gene or polynucleotide sequence for targeting a gene (polynucleotide containing an oligonucleotide complementary to the RNA of the disease related gene), and then to adopt a method for introducing the intended sequence for a target gene or polynucleotide sequence for targeting a gene (polynucleotide containing an oligonucleotide complementary to the RNA of the disease related gene).

When implementing the second method of gene therapy (direct method) and specifically using extracorporeal pre-tests, it is preferable to use in situ PCR or PCR searches for the sequence for a target gene or polynucleotide sequence for targeting a gene to confirm in advance whether or not the intended sequence for the target gene, or polynucleotide sequence for targeting a gene (polynucleotide containing an oligonucleotide complementary to the RNA of the disease related gene) is actually introduced by the gene introduction method; or it is preferable to confirm an increase of the specific activity that is the desired therapeutic effect based on introducing the intended sequence for a target gene or polynucleotide sequence for targeting a gene (polynucleotide containing an oligonucleotide complementary to the RNA of the disease related gene) as well as the proliferation and growth, or proliferation and suppression of the targeted cells. Moreover, if using a viral vector, of course, it is important to confirm the safety of introducing the sequence for a target gene or polynucleotide sequence for targeting a gene during gene therapy by using the PCR method of searching for proliferative retroviruses, by measuring the reverse transcription enzyme activity, or by using the PCR method for monitoring membrane protein (env) genes.

The present invention provides pharmaceutical compositions or pharmaceutical preparations (gene therapy agents), at a pharmaceutically effective amount together with suitable nontoxic pharmaceutical carriers and diluents, containing as the active ingredient: a polynucleotide sequence targeting a gene; a sequence for a target gene; an introduction vector incorporating these sequences; or cells into which the intended sequence for a target gene or polynucleotide sequence for targeting a gene (polynucleotide containing an oligonucleotide complementary to the RNA of the disease related gene) has been introduced.

Examples of pharmaceutical carriers that can be used in the pharmaceutical composition (pharmaceutical preparations) of the present invention include: fillers, extenders, binders, moisteners, disintegrating agents, surfactants, diluents such as lubricants, and excipients as normally used corresponding to the usage form of the preparation; and these may be suitably selected corresponding the dosage unit form of the preparation obtained.

For example, a pharmaceutical preparation containing a vector for introducing a sequence for a target gene, or a polynucleotide sequence for targeting a gene of the present invention is prepared into a form in which the vector is embedded in liposomes, or into a form of culture cells infected by a retrovirus vector containing the desired sequence for a target gene or polynucleotide sequence for targeting a gene.

These may be prepared into a form compounded in phosphate buffer physiological saline (pH 7.4), Ringer's solution, or an injectable agent for an intracellular constituent solution, or can be prepared into a form that can be administered together with substances with a high gene introduction efficiency such as protamine.

The administration method of the pharmaceutical preparation is not particularly limited, and may be decided corresponding to the various preparation forms, to the age, sex and other conditions of the patient, and to the extent of the disease, etc.

The amount of the active ingredient contained in the pharmaceutical preparation as the dosage thereof is not particularly limited, and may be suitably selected in the range corresponding to the desired therapeutic effect, dosing method, treatment period, and the age, sex and other conditions of the patient, etc.

Generally, the dosage of the retrovirus vector containing the desired sequence for a target gene or polynucleotide sequence for targeting a gene as a pharmaceutical preparation may be, for example, from approximately 1x10³ pfu to 1x10¹⁵ pfu as potency of retrovirus per 1 kg body weight per day.

Moreover, cells into which the desired sequence for a target gene or polynucleotide sequence for targeting a gene has been introduced, it is suitable to select from the range of about 1x10⁴ cells/body to 1x10¹⁵ cells/body.

The preparation may be administered once per day or divided into several times per day, and the administrations may be intermittent at from one to several hour intervals. Preferably, substances with a high gene introduction efficacy such as protamine, or preparations containing such, may be coadministered.

Moreover, the cells or tissues of the present invention provide a method for easily testing the changes of function of the target gene as well as a method for suppressing the function of the targeted gene by introducing into the cells or the tissues isolated or purified single strand polynucleotides having continuous components (I) + (II) + (III) to have an RNA function suppression activity of the RNA of genes complementary to the polynucleotide sequence of the previously described component (I) or (III); and also a method for suppressing the expression of RNA or protein of amino acid sequences that are coded by the genes. As indicated in the examples to be described later, with the method the corresponding changes in gene function that appear can be matched and observed by the method for suppressing the function of the target gene based on an RNA function suppression activity of the mRNA of the target gene of the present invention. The functional changes of the gene may be detected by DNA array analysis and Northern blot analysis to determine the expression of the gene, by immunoblot analysis using antibodies to proteins to determine the suppression of the expression of proteins of the amino acid sequences that are coded by the gene, as well as by enzyme response and luminescence response to simultaneously detect changes of various active functions. Thus, the method of the present invention can also provide a method for observing the changes in the functions that cells and tissues have including fluctuations, etc. of the amount of expression of all genes or proteins that are detected in the cells or tissues.

Further, the present invention can provide knockdown cells, tissues, non-human animals, or plants in which the function is reduced in the cells or tissues produced and cultured by the method using the polynucleotide sequence for a target gene of the present invention; and the present invention can provide knockdown cells, tissues, non-human animals, or plants in which the function is reduced in the cells, tissues, non-human animals, or plants produced and cultured by the method for destroying genes of the present invention. Production of non-human knockdown animals is easily achieved by introducing into fertile eggs the sequence for a target gene or the polynucleotide sequence targeting a gene of the present invention, or an introduction vector incorporating these sequences; and such knockdown animals can be produced as mice, rats, rabbits, sheep, pigs, cows, horses, cats, dogs, monkeys, chimpanzees, frogs and fish. In the same way with plants, knockdown plants can be easily produced, for example: legumes such as soybeans and haricots; grains such as rice, wheat, and corn; potatoes; vegetables such as cucumber, tomatoes, bell peppers, egg plant, asparagus, and onions; fruit or tree fruit such as apples, grapes, figs, kiwi, citrus fruit (mandarin oranges, etc.), strawberries, almonds, peaches, melons, and walnuts; ornamental plants such as Japanese cypress, palm, cedar, maple, ferns, gardenia, and periwinkle; and decorative plants such as roses, carnations, chrysanthemum, bellbind, impatiens, and begonias.

By providing the knockdown cells, tissues, non-human animals and plants with the reduced function, it is possible to use the cells or tissues to detect candidate compounds that may have an effect on the function of the cells and tissues. As previously described, if the observation of changes in disease states or in growth or resistance of plants, etc. is linked to changes in the function of cells, tissues, non-human animals or plants, then the effect on cells, and plants, etc. can be observed, and the usefulness of candidate compounds in such fields as drugs and agricultural chemicals can be determined by:
using the sequence for a target gene or a polynucleotide sequence for a target gene of the present invention,
administering candidate compounds to the cells and tissues, and
observing the changes in the function of the genes.

As described above, the present invention can provide a method for detecting candidate compounds that promote destruction of the RNA of target genes by culturing test compounds with cells or tissues, introducing into the cells or tissue an isolated or purified single strand polynucleotide sequence having continuous (I) + (II) + (III) components, and comparing to a control the activity to suppress the RNA function of the RNA of genes complementary to the polynucleotide sequence of the components (I) or (III).

In addition, as a method for detecting the candidate compounds, a method for screening pharmaceutical product target genes using, for example, a polynucleotide sequence for a target gene of the present invention is a screening method for assaying compounds to stimulate or suppress functions of a target gene by introducing an isolated or purified single strand polynucleotide sequence comprising continuous components (I) + (II) + (III) in the cells or tissues, and by determining activity of RNA function suppression activity in relation to mRNA of genes complementary to the polynucleotide sequences of either of the components (I) or (III); and any method selected from the following group may be used as the method for screening pharmaceutical target genes:
(a) using labeling directly or indirectly bound to a candidate compound to measure the binding of the candidate compound and a polypeptide of an amino acid sequence that is coded by the target gene, or a target gene expression product (or a cell or membrane thereof that carries the polypeptide of an amino acid sequence that is coded by the target gene, or a target gene expression product), or a fusion protein thereof;
(b) measuring in the presence of a labeled competition substance the binding of a candidate compound and a cell into which the single strand polypeptide sequence has been introduced (or cells or the membrane thereof carrying the single strand polypeptide sequence), or a fusion substance thereof;
(c) using a detection system applied to a cell or cell membrane carrying a polypeptide of an amino acid sequence that is coded by the target gene or an expression product of the target gene to determine whether or not a candidate compound has a signal produced by suppressing or activating the polypeptide or expression product of the target gene based on the single strand polynucleotide sequence;
(d) preparing a mixture by simultaneously mixing a candidate substance and a solution containing an amino acid sequence that is coded by the target gene or an expression product of the target gene, measuring the activity of the polypeptide or the expression product of the target gene in the mixture, and comparing the activity of the mixture with that of a standard; and
(e) detecting the effect in the cell that the candidate compound has on the mRNA that codes the polypeptide of the amino acid sequence that is coded by the target gene, and on the product of the polypeptide of the amino acid sequence coded by the target gene.

In the above, peptides, proteins, non-peptide compounds, synthetic compounds, fermentation products, cell extract solution, plant extract solution, animal tissue extract solution, and plasma may be cited as examples of the candidate compound, and these compounds may be novel compounds or well-known compounds.

As one example of a concrete method for detecting candidate compounds that promote destruction of RNA of a target gene by using a sequence for a target gene or a polynucleotide sequence for a target gene of the present invention, administering the candidate compound to the cells, issues, or non-human animals, etc. and observing changes of gene function compared to a control: the effect of a candidate compound (influence on measured activity) may be determined by culturing cells or tissue dissolution solution to which the candidate compound is added before and after transfection of the polynucleotide sequence for a target gene indicated in the examples to be described later, and observing the extent of promotion or suppression of the destruction of the target gene by the polynucleotide sequence for a target gene of the present invention before and after.

Regarding the effect of the candidate compound, for example, the influence on the measured activity in the activity test is compared to that of a control or to when the candidate compound is not added, and if promoting approximately 20% or more, preferably approximately 30% or more, and more preferably approximately 50% or more, then the candidate compound may be selected as a compound to promote the activity of the polynucleotide sequence of a target gene of the present invention. On the other hand, for example, the influence on the measured activity in the activity test is compared to that of a control or to when the candidate compound is not added, and if suppressing approximately 20% or more, preferably approximately 30% or more, and more preferably approximately 50% or more, then the candidate compound may be selected as a compound to suppress the activity of the polynucleotide sequence of a target gene of the present invention.

In addition, using the candidate compound obtained by the screening method of the present invention as a drug may be implemented by following ordinary means. For example, pharmaceuticals containing the sequence for a target gene or polynucleotide sequence for a target gene of the present invention, or recombinant vector in which the sequence for a target gene or polynucleotide sequence for a target gene is inserted may be made into a tablet, capsule, elixir, microcapsule, antiseptic solution or suspension solution. Preparations obtained this way are safe and have low toxicity, and therefore may be administered, for example, to mammals (for example, humans, mice, rats, rabbits, sheep, pigs, cows, horses, cats, dogs, monkeys, chimpanzees, etc.). The dosage of the compounds or the salts thereof may differ depending on the target disease, administration target, and administration route. However, if the object is cancer therapy for example, when orally administering a compound wherein the RNA function suppression activity of the RNA of a target gene that is in cancer cells is promoted by the sequence for a target gene or polynucleotide sequence for a target gene of the present invention, or recombinant vector in which the sequence for a target gene or polynucleotide sequence for a target gene is inserted, then generally in adults (body weight 60 kg), approximately 0.1 to 100 mg, preferably approximately 1.0 to 50 mg, and more preferably approximately 1.0 to 20 mg of the compound is administered daily. If administered non-orally, the single dose of the compound differs depending on the administration target and the targeted disease. However, if the object is cancer therapy for example, generally to adults (body weight 60 kg), when administering a compound in the form of an injectable agent wherein the RNA function suppression activity of the RNA of a target gene that is in cancer cells is promoted by the sequence for a target gene or polynucleotide sequence for a target gene of the present invention, or recombinant vector in which the sequence for a target gene or polynucleotide sequence for a target gene is inserted, then approximately 0.01 to 30 mg, preferably approximately 0.1 to 20 mg, and more preferably approximately 0.1 to 10 mg of the compound is administered daily by intravenous injection. The same applies with other animals.

In addition, it is possible to provide an animal with the target gene destroyed by using the sequence for a target gene or polynucleotide sequence for a target gene of the present invention, or recombinant vector in which the sequence for a target gene or polynucleotide sequence for a target gene is inserted. Specifically, the present invention provides a non-human mammal having the sequence for a target gene or polynucleotide sequence for a target gene, or recombinant vector in which the sequence for a target gene or polynucleotide sequence for a target gene is inserted; and the non-human mammal is a rodent, and the rodent is a mouse or a rat.

In the present invention, the non-human mammal having the sequence for a target gene or polynucleotide sequence for a target gene, or recombinant vector in which the sequence for a target gene or polynucleotide sequence for a target gene is inserted, can be produced by using the calcium phosphate method, electro-pulse method, the lipofection method, the agglutination method, the micro-injection method, the particle gun method, or the DEAE-dextran method, etc. to transplant the intended polynucleotide sequence targeting a gene, sequence for a target gene, or recombinant vector inserted into which is the sequence for a target gene or polynucleotide sequence for targeting a gene into an unfertilized egg, fertilized egg, sperm, or germinal cell containing primordial cells thereof, preferably in the stage of embryonic development in the generation of the non-human mammal (more preferably, at the stage of a single cell or fertilized egg cell and generally prior to the 8 cell period). Moreover, depending on the method for transplanting the polynucleotide sequence targeting a gene, sequence for a target gene, or recombinant vector inserted into which is the sequence for a target gene or polynucleotide sequence for targeting a gene, it is possible to transplant the intended polynucleotide sequence targeting a gene, sequence for a target gene, or recombinant vector inserted into which is the sequence for a target gene or polynucleotide sequence for targeting a gene into corporeal cells, living organs, and tissue cells, and to use these in cell cultures and tissue cultures, etc. Further, it is possible to produce an animal with transplanted polynucleotide sequence targeting a gene, sequence for a target gene, or recombinant vector inserted into which is the sequence for a target gene or polynucleotide sequence for targeting a gene of the present invention by fusing these cells with the previously described germinal cell using independent or well-known cell fusion methods.

For example, cows, pigs, sheep, goats, rabbits, dogs, cats, guinea pigs, hamsters, mice, and rats may be used as the non-human mammals. Of these, an animal with a comparatively short individual generation and biological cycle is preferable in terms of creating disease animal models, and rodents that are easily propagated, especially mice (examples of pure lines include the C57BL/6 strain and the DBA2 strain; and examples of crossbred lines include the B6C3F1 strain, the BDF1 strain, the B6D2F1 strain, the BALB/c strain, and the ICR strain), or rats (for example, Wistar, SD, etc.) are preferred. In addition, knockdown pigs with suppressed genes related to organ rejection reactions, etc. are preferable when transplanting organs to humans.

The present invention can provide a single strand polynucleotide having an activity to suppress the function the RNA of a target gene, as well as a method for suppressing and controlling the functional expression of the target gene thereof; and by selectively suppressing the functional expression of the gene, the present invention can provide a single strand polynucleotide having an activity to suppress the function of the targeted protein or RNA, as well as a method for suppressing and controlling the functional expression thereof. In addition, the present invention can provide a simple method for analyzing the functions of genes, a screening method for pharmaceutical product target genes, a method for evaluating in vivo pharmaceutical product target genes prepared by knockdown mice, and pharmaceutical compositions for genetic diseases (gene therapy agents).

### BEST MODE FOR CARRYING OUT THE INVENTION

Examples will be cited below in order to explain the present invention in further detail.

### Example 1: Synthesis of polynucleotide for a target gene of the present invention

In the present example, the polynucleotides for a target gene having two types of component sequences were synthesized taking the sequence of 19 nucleotides corresponding to sequence positions 434 to 452 of the luciferase gene (Genbank Accession No. U47296), the target gene, as the target site for RNA interference.

The RNA sequence was synthesized using a commercial automatic synthesizer (ABI3900 high throughput DNA synthesizer manufactured by Applied Biosystems) and reagents for RNA synthesis using the phosphoroamidite method. Further, RNA of a forward sequence (F) comprising a 21mer complementary to sequence positions 936 to 954 of EGFP (Genbank Accession No. U55763) and a lipase sequence (R) were synthesized as the non-specific controls.

Of the polynucleotides for a target gene of the present invention thus obtained, a sequence for component (II) with 12 bases was named uGL3.12RNA, and a sequence for component (II) with 7 bases was named uGL3.7RNA. The sequences of the synthesized uGL3.12RNA and uGL3.7RNA are indicated in SEQ ID Nos. 1 and 2 respectively, and the component sequences of uGL3.12RNA and uGL3.7RNA are indicated in SEQ ID Nos. 3 and 4.

Moreover, as the non-specific controls, the forward sequence (F) RNA comprising a 21mer complementary to sequence positions 936 to 954 of EGFP, and lipase sequence (R) RNA are as indicated in SEQ ID Nos. 5 and 6, and 2 basses of UU (uracil) were added respectively to the terminals of the sequences.

### Example 2: Test of RNA function suppression activity using a polynucleotide for a target gene of the present invention

### 1. Preparation of RNA for RNA transfection

100-picomole/µL solutions were prepared by dissolving the uGL3.12RNA (142 nanomole) and uGL3.7RNA (135 nanomole) obtained above respectively in distilled water.

Next, mixed solutions of 30 µL of the RNA solution, 30 µL of distilled water, and 240 µL of buffer solution (100 M potassium acetate, 30 mM HEPES-KOH adjusted to pH 7.4, 2 mM magnesium acetate) was prepared, and were taken to be the uGL3.12RNA source solution and the uGL3.7RNA source solution (source solution: 10 pmole/uL). Dilutions corresponding the dilution magnitudes (x5, x50) were all prepared with the buffer solution.

### 2. Preparation of the non-specific control siRNA

Ten picomoles each of the single strand forward sequence (F) RNA comprising a 21mer complementary to sequence positions 936 to 954 of EGFP, and lipase sequence (R) RNA obtained in aforementioned Example 1 were mixed, and an annealing buffer was added to make 100 µL. This solution was diluted 5 times with buffer solution and was taken to be the control siRNA solution (EGFPc2).

### 3. Preparation of transfection composite

For the preparation of a reporter gene expression vector, a transfection composite was prepared by adding the various RNA prepared above respectively to cloning vector pGL3-control (manufactured by Promega) having luciferase reporter genes, and to pRL/TK (manufactured by Promega) having (Renilla) luciferase genes.

Specifically, a solution (EGFPc2) (solution a) was prepared by adding the reporter gene (1µg of pGL3-control and 0.1 µg of pRL/TK) and the polynucleotides for a target gene of the present invention uGL3.12RNA, uGL3.7RNA, or non-specific control siRNA to 50 µL of OPTI-MEM non-serum medium (manufactured by GIBCO-BRL).

Separately, a suspension was made by adding 1.5 µL of lipofectamine 2000 (manufactured by Life Technology) to 50 µL of OPTI-MEM medium, and leaving to stand for 5 minutes at room temperature (solution b). Then, solutions a and b were mixed, and allowed to react for 20 minutes to make the transfection composite.

### 4. Transfection

First, up until immediately before confluence, HeLa3 cells were pre-cultured at 37° C under 5% CO₂ in a 10-cm diameter petri dish using D-MEM medium (Sigma) to which was added DMEM/10 medium (10% bovine fetal serum (manufactured by INTERGEN, #1020-90)). After rinsing the pre-cultured cells with PBS, the cells were detached by treating with 1 mL of trypsin-EDTA, and resuspended in 10 mL of DMEM/10. The number of cells was counted using a hemocyte counting plate, and adjusted using DMEM/10 to make 1.2x10⁵/0.5 mL. 0.5 mL of HeLa3 cells were added to each well of a 24-well plate, and cultured for 24 hours at 37° C under 5% CO₂.

For the transfection, the culture supernatant of the HeLa3 cells in the 24-well plate was removed, and 0.5 mL of new DMEM/10 was added to each well. Next, after adding 0.1 mL transfection composite to each well, and after making uniform by gently shaking the plate, [the cells] were cultured for 24 hours at 37° C under 5% CO₂.

### 5. Measuring the activity of luciferase

The Dual-Luciferase Reporter Assay System: #E1910 (manufactured by Promega) was used, and the procedures in the attached protocol were followed for this reagent. 400 µL of the lysis solution attached to the kit was added to the culture plate, and after dissolving the cells and centrifuging for 5 seconds at 1500 rpm with a tabletop centrifuge, 20 µL of this supernatant was added to 1 µL of the attached reaction reagent LARII (100µL), and the measurement of the first chemiluminescence with a luminometer was conducted using the GENios (TECAN) emission measurement program.

The measured values of firefly luciferase were corrected with the values of (Renilla) luciferase (internal standard), and taking the values without the presence of RNA as 1.0, the relative values of F-Luc/R-Luc were calculated.

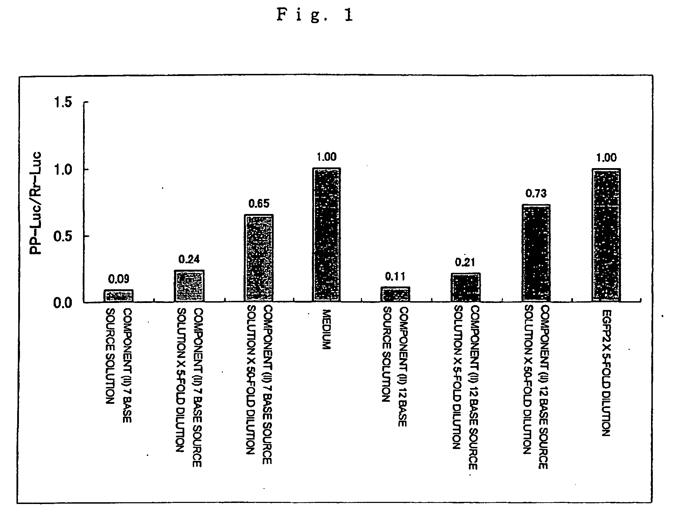

The results are indicated in Fig. 1 or Table 1.

**Table 1**

| | PP-Luc | Rr-Luc | PpRr corrected value | |
|---|---|---|---|---|
| Component (II) 7 base source solution | 3004 | 70136 | 0.04 | 0.09 |
| Component (II) 7 base source solution x 5 fold dilution | 25367 | 223084 | 0.11 | 0.24 |
| Component (II) 7 base source solution x 50 fold dilution | 100749 | 320428 | 0.31 | 0.65 |
| Medium | 149861 | 310559 | 0.48 | 1.00 |
| Component (II) 12 base source solution | 3726 | 72009 | 0.05 | 0.11 |
| Component (II) 12 base source solution x 5 fold dilution | 31507 | 304565 | 0.10 | 0.21 |
| Component (II) 12 base source solution x 50 fold dilution | 108888 | 309057 | 0.35 | 0.73 |
| EGFP2 x 5 fold dilution | 112486 | 233487 | 0.48 | 1.00 |

As indicated in Fig. 1 or Table 1, when regarding the test results in corrected values, and making the comparison by taking the non-specific sequence control as 1.00, the values for the 50 fold dilution, 5 fold dilution and source solution of the component (II) with a base length of 7 were 0.65, 0.24, and 0.09 respectively indicating a concentration-dependent decrease, and approximately 90% of the activity was suppressed by the source solution. Moreover, the values for the 50 fold dilution, 5 fold dilution and source solution of the component (II) with a base length of 12 were 0.73, 0.21, and 0.11 respectively, likewise indicating a concentration-dependent decrease with approximately 90% of the activity suppressed by the source solution. Thus it was demonstrated that in both, irrespective of the size of the component (II), the luciferase activity was suppressed in a concentration-dependent manner.

From the results of Fig. 1 or Table 1, it was confirmed that uGL3.12RNA and uGL3.7RNA, the single strand polynucleotides for a target gene of the present invention, suppressed luciferase activity, which is the target gene, in a concentration-dependent manner, and have an RNA function suppression activity.

In addition, no difference in activity based on the length of the component (II) (base length of 7 or base length of 12) was observed in this test.

Continuing, it was confirmed (data not indicated) that even when using a hairpin-like complementary sequence (-AATT-), the component (II) has an RNA suppression activity.

### Example 3: Effect to suppress RNA function using a Lamin A/C gene function suppression vector

In this example, a vector to be expressed inside the cell was produced taking the sequence of 23 nucleotides corresponding to sequence positions 640 to 662 of the Lamin A/C gene (Genbank Accession No. X03445), the target gene, as the target site for RNA interference.

### (1) Preparation of the Lamin A/C gene function suppression cells

1. Preparation of the Lamin A/C gene function suppression vector:
   A Lamin A/C gene function suppression vector was prepared as follows.
   Human U6 promoter was amplified by PCR using the following primers (oligomer 1 and 2; SEQ ID No. 7, 8). Taking this PCR amplification fragment as a template, PCR was conducted using oligimer-1 (SEQ ID No. 7) and oligomer 3 (SEQ ID No. 9), and altered U6 promoter was prepared with an introduced restriction enzyme *Csp*45I cleavage site. This amplification fragment was cleaved by the restriction enzymes *Eco*RI and *Xba*I, and was cloned to pUC19 vector likewise cleaved by the restriction enzymes *Eco*RI and *Xba*I (pUC19U6).
   After mouse H1 promoter was amplified by PCR using the following promoters (oligomers 4 and 5; SEQ ID Nos. 10, 11), the amplified fragments were cleaved by the restriction enzymes *Eco*RI and *Sal*I. This DNA fragment was cloned to pUC19 vector cleaved by the restriction enzymes *Eco*RI and *Sal*I (pUC19H1).
   Oligomer name 1 (SEQ ID No. 7): U6P.F, within box; EcoRI cleavage site TCTTTGGAATTCAAGGTCGGGCAGGAAGAGGGCCTA Oligomer name 2 (SEQ ID No. 8): U6CSP45R, within box; Csp45I cleavage site TGCTGCCGAAGCGAGCACGGTGTTTCGAACTTTCCACAAG Oligomer name 3 (SEQ ID No. 9): U6P.R, within box; XbaI cleavage site AAAAATTCTAGATGTAAAAATAGTGTTGTGTGCCTAGGATATGTGCTGCCGAAGCGAGCAC Oligomer name 4 (SEQ ID No. 10): mHl.F, within box; *Eco*RI cleavage site TTTTTGAATTCATGCAAATTACGCGCTGTG Oligomer name 5 (SEQ ID No. 11): mH1.R, within box; *Sal*I cleavage site) CCAAGGGTCGACAAAAAGATATCTGGATCCGTCTAGACCGGCCGCCACTATAAG
   Next, after annealing oligomer 1 (SEQ ID No. 12) and oligomer 2 (SEQ ID No. 13), synthesizing DNA with Ex-Taq (Takara), cleaved with *Xba*I and *Sal*I, and then cloned to pUC19H1 vector similarly cleaved with *Xba*I and *Sal*I and named pUC19H1Lamin.
   Moreover, a pUC19-neo vector was prepared by inserting neomycin-resistant gene amplified from pcDNA3.1(+) vector (Invitrogen) by the following primers (oligomers 5 and 6, SEQ ID Nos. 16, 17) into a site of the restriction enzyme NdeI of the pUC19 vector. , a fragment having RNA suppression function cleaved with *Eco*RI, *Hind*III from the pUC19H1-Lamin vector was inserted into the *Eco*RI, *Hind*III cleavage site of this vector to make a Lamin A/C gene function suppression vector.
   Oligomer name 1 (SEQ ID No. 12): hLaminAC-4.H1F, within box; XbaI cleavage site, underlined; stem region, italic; loop region AAAGGTCTAGACCCTAAAGAATAAGTCTTCTCCAGCTCCTTAAGGAG Oligomer name 2 (SEQ ID No. 13): hLaminAC-4.R, within box; *Sal*I cleavage site, underlined; stem region, italic; loop region ATTATGTCGACAAAAAGAATAAGTCTTCTCCAGCTCCTTAAGGAG Oligomer name 3 (SEQ ID No. 14): Neo-Nde.F, within box; *Nde*I cleavage site, underlined; stem region italic; loop region AGAATTCCATATGTGGAATGTGTGTCAGTTAG Oligomer name 4 (SEQ ID No. 15): Neo-Nde.R, within box; *Nde*I cleavage site AGAATTCCATATGAGGTCGACGGTATACAGAC
2. Preparation of transfection composite: 1 µg of the Lamin A/C gene function suppression vector obtained in 1 above was added to 50 µL of OPTI-MEM (manufactured by Invitrogen) (solution A). Separately, 3 µL of lipofectamine 2000 (manufactured by Invitrogen) was added to and mixed with 50 µL of OPTI-MEM, and left to stand at room temperature for 5 minutes (solution B). The transfection composite was prepared by mixing solutions A and B, and allowing to react for 20 minutes at room temperature.
3. Transfection: The day prior to transfection, a 2x10⁵/mL solution of HeLa S3 cells was prepared using DMEM medium (manufactured by Sigma) to which 10% FBS was added, and 0.5 mL was added per well in a 24-well plate. The cells were cultured overnight at 37° C under 5% CO₂ in a CO₂ incubator (manufactured by Sanyo). After 100 µL of the transfection mix prepared in 2 above was added per well of the 24-well plate, the culture plate was returned to the CO₂ incubator, and culture was continued overnight.
4. Sorting and establishment of cell line: After rinsing each well of the cell culture plate with PBS, the cells were detached with trypsin EDTA solution (manufactured by Invitrogen), and recovered. The recovered cells were resuspended in 10 mL of DMEM medium (manufactured by Sigma) supplemented with 10% FBS, and the full amount was dispersed in a 10 cm dish. The following day, the culture supernatant was aspirated and removed, and 10 mL of DMEM medium (manufactured by Sigma) containing section culture solution (10%FBS, 500 µg/mL of geneticin (manufactured by Invitrogen)) was added. The cells were cultured at 37° C under 5% CO₂ in a CO₂ incubator (manufactured by Sanyo) while replacing the selection culture solution with fresh solution once every 2 to 3 days, and finally 31 geneticin-resistant cell lines were obtained. The cell lines obtained were used as Lamin A/C gene function suppression cell lines in the following evaluations.

### (2) Evaluation of Lamin A/C gene function suppression cell lines

### 1. RNA preparation:

After 10-cm dishes in which the 31 types of Lamin A/C gene function suppression cell lines were cultured were rinsed with PBS two times, the cells were lysed by adding 3 mL of TRIZOL (manufactured by Invitrogen) per dish. Each of the cell lysate were transferred to individual 15-mL centrifuge tubes, 0.2 mL of chloroform was added to each and mixed vigorously, and after leaving to stand for 3 minutes at room temperature, the solutions were centrifugally separated for 40 minutes at 3,000 rpm at 4° C. After transferring the respective liquid phases (the highest parts) to new tubes, 0.5 mL of isopropanol was added, and left to stand for 5 to 10 minutes at room temperature. After centrifugally separating for 40 minutes at 3,000 rpm 4° C, the RNA precipitate was taken, rinsed one time with 70% ethanol, and dissolved in DW. The absorbance of the various samples at 260 nm was measured with a DU650 (manufactured by Beckman), the RNA concentrations were calculated based on the measured values, and used for Northern analysis.

### 2. Preparation of protein

After 10-cm dishes in which 31 types of Lamin A/C gene function suppression cell lines were cultured were rinsed with PBS, the cells were detached with trypsin EDTA solution (manufactured by Invitrogen), and recovered. The recovered cells were rinsed with PBS again, and the cells were recovered by micro-centrifuge for 5 minutes at 5,000 rpm. The recovered cells were dissolved in boiling lysis buffer (5% SDS, 5 mM sodium ortho-vanadate, 50 mM Tris pH 7.4), then treated for 5 minutes at 100° C, and then immediately ice-cooled. Centrifugation was conducted for 20 minutes at 15,000 rpm 4° C, and the supernatant was recovered and taken to be the protein solution. After the protein solution was diluted 10 fold using distilled water, the total protein concentration was measured. The absorbance of the dilute protein solution after allowing to react with Advanced Protein Assay Reagent (manufactured by Cytosleleton) was measured using a GENios (manufactured by TECAN). Taking BSA as the standard product, the measured values were corrected, and samples were made by adjusting to a final concentration of 1µg/µL.

### 3. Analysis of Lamin A/C mRNA expression using Northern hybridization

The expression of Lamin A/C mRNA was analyzed using the RNA prepared in 1. above.

### Lamin A/C mRNA expression analysis

After separating 20 µg of the total RNA by electrophoresed in denatured agarose gel, transfer to a Hybond-N+ membrane (Amersham Pharmacia Biotech) was conducted by the capillary blotting method. The RNAs were then crosslinked to the membrane by UV crosslinking method. After treatment for 1 hour at 42° C with a pre-hybridization solution (50% formamide, 5xSSPE, 2xDenhardt's solution, 0.1% SDS, 100 µg/mL denatured salmon sperm DNA), the membrane was hybridized overnight using lamin A/C gene fragment isotope labeled with ³²PdCTP as a probe. The membrane was rinsed for 5 minutes at room temperature using a 2xSSC/0.1% SDS solution, and this procedure was repeated 3 times. Subsequently, the membrane was rinsed two times for 10 minutes at 50° C and for 10 minutes at 65° C. When rinse was finished, the membrane was exposed overnight to X-ray film.

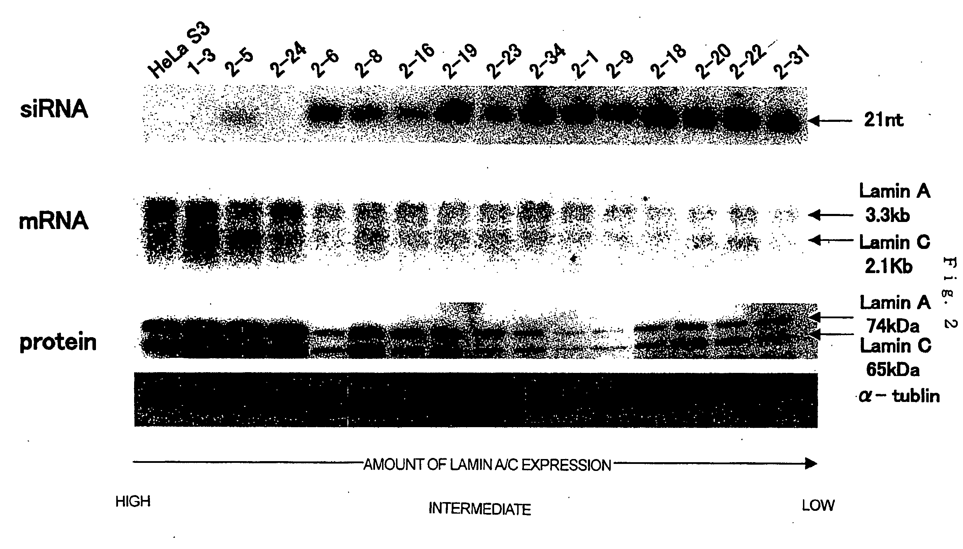

The results are indicated in Fig. 2. Compared to the cell lines in which the vectors were not introduced, the Lamin A/C gene function suppression cell lines suppressed the expression of Lamin A/C mRNA, and it was demonstrated that Lamin A/C gene function suppression vectors have a gene specific RNA function suppression activity.

### 4. Analysis by the Western method

The concentration-adjusted protein samples in 2. above were diluted 2 times using SDS buffer. 6 µL thereof was laminated on 13% SDS acrylamide gel, and SDS PAGE was conduced for 80 minutes under 40 mA and 300 V conditions. Using a semi-dry blotting device, transfer of the protein from the electrophoresed gel to a nitrocellulose membrane (BIORAD) was conducted under conditions of 300 V, 140 mA, 60 min. The nitrocellulose membrane with blotted proteins was treated with 1000-fold diluted anti-Lamin A/C antibody (manufactured by BD Bioscience) or control antibody (αTubulin antibody, Zymed Laboratories), and then allowed to react with HRP labeled goat anti-mouse IgG antibody. The Lamin A/C specific bands were detected by allowing the nitrocellulose membrane after antigen antibody reaction to react with ECL western blotting detection reagent (Amersham Bioscience), and exposing to X-ray film.

The results are indicated in Fig. 2. The concentrations of the bands detected for the various samples closely agreed with the Lamin A/C mRNA concentrations in the Northern analysis, and it was demonstrated that not only mRNA, but also protein after translation were suppressed.

### Example 4. RNA function suppression effect of firefly luciferase gene function suppression vector

In this example, a firefly luciferase gene function suppression vector that expresses the polynucleotide for a target gene inside the cell was prepared by taking the 25 nucleotide sequence equivalent to sequence positions 357 to 381 of the firefly luciferase gene (Genbank Accession No. 47296), the target gene, as the RNA interference target site; and the RNA function suppression effect thereof was evaluated.

Preparation of firefly luciferase gene function suppression vectors: Under the control of U6 promoter and H1 promoter, two types of vectors (U6H1 25stem-Luc, and U6H1 25stem loop-Luc) that express the polynucleotide to suppress firefly luciferase gene function, as well as a control vector, which did not contain a sequence in relation to the firefly luciferase gene, were constructed.

### Control vector

The pUC19U6 prepared in Example 3 was cleaved with restriction enzymes EcoRI and SalI to isolate human U6 promoter, and the fragments thereof were cloned to pBS vector cleaved with EcoRI and SalI (pBSU6). Next, pUC19H1 vector prepared in Example 3 was amplified by PCR with the following primers (oligomers 1 and 2) to isolate mouse H1 promoter, the amplified fragments thereof were cleaved with SacI and SpeI, cloned to pBSU6 cleaved by SacI and SpeI, and taken as the control vector.
Oligomer name 1: biHlpro.SacI Sequence No. 16 TTTTTGAGCTCATGCAAATTACGCGCTGTG Oligomer name 2: biH1pro.SpeI-2 SEQ ID No. 17

TTTTTACTAGTTGGTCTAGACCGGCCGCCAC

### Luciferase gene RNA function suppression vector

### 1. U6H1 25stem-Luc

Oligomer 1 and oligomer 2, SEQ ID Nos. 18 and 19, below were annealed, and cloned to the control vector cleaved by the restriction enzymes Csp45I and XbaI.
Oligomer name 1 SEQ ID No. 18: 25s#4F in box; Csp45I cleavage terminal, underlined stem region CGAAAAAAAGCATAAGGCTATGAAGAGATACGCCTTTTTGGT Oligomer name 2 SEQ ID No. 19: 25s#4R in box; XbaI cleavage terminal, underlined stem region

CTAGACCAAAAAGGCGTATCTCTTCATAGCCTTATGCTTTTTTT

### 2. U6H1 25stem loop-Luc

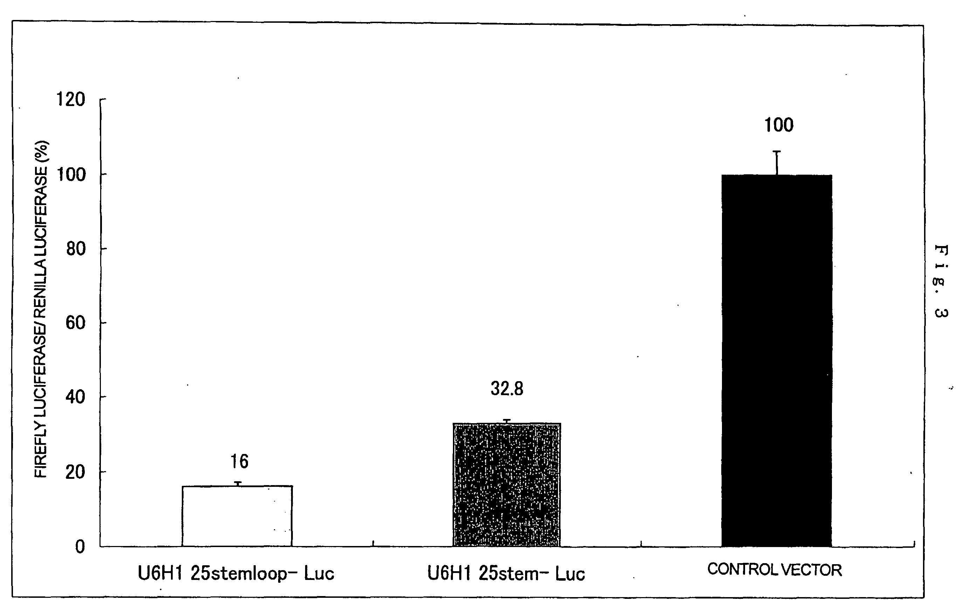

Oligomer 1 and oligomer 2, SEQ ID Nos. 20 and 21, below were annealed, DNA synthesis was conducted using Ex-Taq, and double strand oligo was synthesized. The synthesized double strand oligo was cleaved with the restriction enzymes Csp45I and XbaI, and was cloned to pBSU6 cleaved with Csp45I and XbaI.
Oligomer name 1;ST-UHGL3#4F SEQ ID No. 20; in box, Csp45I cleavage site; underlined, stem region; italics, loop region 
TGGAAAGTTCGAAAAAAGCATAAGGCTATGAAGAGATACGCCTTAAGGCGT
(strand length 52mer)
 Oligomer name 2 SEQ ID No. 21;ST-UHGL3#4R, in box, XbaI cleavage site; underlined, stem region; italics, loop region 
CACGGGTCTAGACCAAAAGCATAAGGCTATGAAGAGATACGCCTTAAGGCGT

(strand length 53mer)
1. Preparation of transfection composite: 1.8 µg of pGL3 control vector (manufactured by Invitrogen), 0.2 µg or pRL/TK vector (manufactured by Invitrogen), and 20 µg of RNAi induction vector obtained in 1 above were added to 200 µL of OPTI-MEM (manufactured by Invitrogen) (solution A). Solutions A were individually prepared for a total of 3 types of RNAi induction vector and control vector. Separately, 3 tubes prepared by adding and mixing 12 µL of lipofectamine 2000 (manufactured by Invitrogen) with 200 µL of OPTI-MEM, and allowing to stand at room temperature for 5 minutes (solution B). Transfection composite (3 types) was prepared by mixing solution A and solution B, and allowing to react at room temperature for 20 minutes.
3. Transfection: The day prior to transfection, a 10⁵/mL solution of HeLa S3 cells was prepared using DMEM medium (manufactured by Sigma) to which 10% FBS was added, and 0.5 mL was added per well in a 24-well plate. The cells were cultured overnight at 37° C under 5% CO₂ in a CO₂ incubator (manufactured by Sanyo). After 100 µL of the 3 types of transfection composite prepared in 2 above was added per well of the 24-well plate (4 wells for one type; n=4), the culture plate was returned to the CO₂ incubator, and culturing was continued overnight.
3. Luciferase assay: The Dual-Luciferase Reporter Assay System (manufactured by Promega) was used, and the procedures in the protocol attached to the kit were followed. The medium was aspirated and removed from the culture plate, and the cells were rinsed once with PBS. After 0.2 mL of the lysis solution attached to the kit was added per well, and the cells were lysed, 20 µL of the lysed solution was mixed with 1 µL of the attached reaction reagent LAEII (100µL), and the firefly luciferase activity was measured using the GENios (TECAN) emission measurement program. Continuing, after adding 100 µL of Stop & Gro reagent, the activity of the Renilla luciferase was measured by the GENios emission measurement program. After calculating the values by dividing the firefly luciferase measured values by the Renilla luciferase measured values, the respective relative values were calculated taking the value obtained by the control vector as 100.
4. Analytic results: The results are indicated in Fig. 3. When treating with various RNAi induction vector, the amount of luciferase expression when treated with the control vector taken to be 100, and the expression was reduced 32.8% with U6H1 25stem-Luc, and 16% with U6H1 25stemloop-Luc. The suppression of luciferase activity was demonstrated with both function suppression vectors. The results in Fig. 3 confirm that the function suppression vectors of the present invention effectively suppress the activity of luciferase, the target gene, and have an RNA function suppression activity.

## Claims

1. A polynucleotide sequence which is a polynucleotide sequence for a target gene comprising an isolated or purified single strand polynucleotide sequence comprising continuous components (I) + (II) + (III) wherein
the polynucleotide sequence for a target gene has an RNA function suppression activity in relation to RNA having a sequence complementary to either the component (I) or (III) or a partial sequence thereof,
where, the component (III) comprises a continuous polynucleotide sequence of 15 to 30 that has a sequence complementary to the target gene,
the component (IT) is a nucleotide sequence or non-nucleotide sequence with a base length of from 0 base to 10 kilobases (where, 0 base means a bond), and
the component (I) is a polynucleotide sequence comprising a sequence complementary to the component (III).

2. The polynucleotide sequence according to claim 1 wherein the polynucleotide sequence of the component (III) comprises DNA or RNA.

3. The polynucleotide sequence according to claim 1 wherein the component (I) or (III) further has a sequence comprising from 1 to several U, T, G, C, or A bases on at least one terminal, or has deleted, substituted or added inside of the complementary sequence.

4. The polynucleotide sequence according to claim 1 wherein the polynucleotide sequence is obtained by chemical synthesis or gene recombination technology.

5. A polynucleotide sequence for a target gene comprising single strand RNA of SEQ ID No. 1 or 2.

6. The polynucleotide sequence according to claim 1 wherein the component (II) is a nucleotide sequence or a non-nucleotide sequence, or a combination thereof.

7. The polynucleotide sequence according to claim 6 wherein the nucleotide sequence of the component (II) comprises a nucleotide sequence of 1 base or more and less than 10 kilobases.

8. The polynucleotide sequence according to claim 7 wherein the nucleotide sequence of the component (II) comprises a nucleotide sequence of a length of from 1 base to several hundred bases.

9. The polynucleotide sequence according to claim 8 wherein the nucleotide sequence of the component (II) comprises a nucleotide sequence of a length of from 1 base to several dozen bases.

10. The polynucleotide sequence according to claim 9 wherein the nucleotide sequence of the component (II) comprises a nucleotide sequence of a length of from 1 base to 20 bases.

11. The polynucleotide sequence according to claim 10 wherein the component (II) is indicated by SEQ ID No. 3 or 4.

12. The polynucleotide sequence according to claim 1 wherein the nucleotide sequence or non-nucleotide sequence of the component (II) comprises PNA, a cytoplasm translocation sequence, a sequence having a decoy activity, an interferon induction suppressing sequence, a sequence having any of RNase suppression activity, antisense activity, ribozyme activity, or transfer RNA, or a combination of these.

13. A method for manufacturing the polynucleotide sequence of any of claims 1 to 12 by chemical synthesis or gene recombination technology.

14. A recombinant vector wherein the polynucleotide sequence for a target gene of any of claims 1 to 12 is inserted in a vector.

15. A method of manufacturing the recombinant vector of claim 14 wherein the polynucleotide sequence for a target gene of any of claims 1 to 12 is inserted in a vector.

16. A method for screening pharmaceutical product target genes using the polynucleotide sequence for a target gene of any of claims 1 to 12, which is a screening method for assaying compounds to stimulate or suppress functions related to a target gene by introducing an isolated or purified single strand polynucleotide sequence comprising continuous components (I) + (II) + (III) in cells or tissues, and using a single strand polynucleotide sequence to increase or decrease the RNA function suppression activity of genes having a sequence complementary to the polynucleotide sequences of either of the component (I) or (III); wherein the method for screening pharmaceutical product target genes employs any one method selected from the following methods:
(a) using labeling directly or indirectly bonded to a candidate compound to measure the binding of the candidate compound and a polypeptide of an amino acid sequence that is coded by the target gene, or a target gene expression product (or a cell or membrane thereof that carries the polypeptide of an amino acid sequence that is coded by the target gene, or a target gene expression product), or a fusion protein thereof;
(b) measuring in the presence of a labeled competition substance the binding of a candidate compound and a cell into which the single strand polypeptide sequence has been introduced (or cells or the membrane thereof carrying the single strand polypeptide sequence), or a fusion substance thereof;
(c) using a detection system applied to a cell or cell membrane carrying a polypeptide of an amino acid sequence that is coded by the target gene or an expression product of the target gene to determine whether or not a candidate compound has a signal produced by suppressing or activating the polypeptide or expression product of the target gene based on the single strand polynucleotide sequence;
(d) preparing a mixture by simultaneously mixing a candidate substance and a solution containing an amino acid sequence that is coded by the target gene or an expression product of the target gene, measuring the activity of the polypeptide or the expression product of the target gene in the mixture, and comparing the activity of the mixture with that of a standard; and
(e) detecting the effect in the cell that the candidate compound has on the mRNA that codes the polypeptide of the amino acid sequence that is coded by the target gene, and on the product of the polypeptide of the amino acid sequence coded by the target gene.

17. A pharmaceutical composition taking the polynucleotide sequence for a target gene according to any of claims 1 to 12 as the active ingredient.

18. A pharmaceutical composition taking the recombinant vector of claim 14 as the active ingredient.

19. A method for introducing an isolated or purified single strand polynucleotide sequence comprising continuous components (I) + (II) + (III) into cells or tissues, and to suppress the function of a target gene based on an RNA function suppression activity of a gene having a sequence complementary to the polynucleotide sequence of either of the component (I) or (III),
where, the component (III) comprises a continuous polynucleotide sequence of 15 to 30 that has a sequence complementary to the target gene,
the component (II) is a nucleotide sequence or non-nucleotide sequence with a base length of from 0 base to 10 kilobases (where, 0 base means a bond), and
the component (I) is a polynucleotide sequence comprising a sequence complementary to the component (III).

20. The method according to claim 19 wherein the nucleotide sequence comprising polynucleotides of the component (III) comprises DNA or RNA.

21. The method according to claim 19 wherein the component (I) or (III) is DNA or RNA that has a sequence comprising from 1 to several of U, T, G, C, or A bases on any terminal, or has deleted, substituted or added inside.

22. The method according to claim 19 wherein the polynucleotide sequence is obtained by chemical synthesis or gene recombination technology.

23. The method according to claim 19 wherein the single strand polynucleotide sequence comprises a single strand RNA of SEQ ID No. 1 or 2.

24. The method according to claim 19 wherein the component (II) is a nucleotide sequence or a non-nucleotide sequence, or a combination thereof.

25. The method according to claim 24 wherein the nucleotide sequence of the component (II) comprises a nucleotide sequence of 1 base or more and less than 10 kilobases.

26. The method according to claim 25 wherein the nucleotide sequence of the component (II) comprises a nucleotide sequence of a length of from 1 base to several hundred bases.

27. The method according to claim 26 wherein the nucleotide sequence of the component (II) comprises a nucleotide sequence of a length of from 1 base to several dozen bases.

28. The method according to claim 27 wherein the nucleotide sequence of the component (II) comprises a nucleotide sequence of a length of from 1 base to 20 bases.

29. The method according to claim 28 wherein the component (II) is indicated in SEQ ID No. 3 or 4.

30. The method according to claim 18 wherein the nucleotide sequence or non-nucleotide sequence of the component (II) comprises PNA, a cytoplasm translocation sequence, a sequence having a decoy activity, an interferon induction suppressing sequence, a sequence having any of RNase suppression activity, antisense activity, ribozyme activity, or transfer RNA, or a combination of these.

31. A method for suppressing expression of the protein of a target gene based on the method for suppressing the function of a target gene according to claims 19 to 30.

32. A method for suppressing the activity of a transcript of a target gene based on the method for suppressing the function of a target gene according to claims 19 to 30.

33. A knockdown cell or tissue or a non-human knockdown animal or a knockdown plant produced and cultured by the method of claim 31 or 32.

34. A knockdown cell or tissue or a non-human knockdown animal according to claim 33 that is for organ transplants.

35. A gene therapy agent comprising a pharmaceutical composition according to claim 17 or 18.

36. A method for testing the function of a target gene by introducing an isolated or purified single strand polynucleotide sequence comprising continuous components (I) + (II) + (III) into cells, tissues, non-human animals, or plants to have an RNA function suppression activity of a gene having a sequence complementary to the polynucleotide sequence of either of the component (I) or (II),
where, the component (III) comprises a continuous polynucleotide sequence of 15 to 30 that has a sequence complementary to the target gene,
the component (II) is a nucleotide sequence or non-nucleotide sequence with a base length of from 0 base to 10 kilobases (where, 0 base means a bond), and
the component (I) is a polynucleotide sequence comprising a sequence complementary to the component (III).

37. A method for detecting a candidate compound to reinforce the function of a target gene comprising the steps of:
introducing an isolated or purified single strand polynucleotide sequence comprising continuous components (I) + (II) + (III) into cells, tissues, non-human animals, or plants after culturing the test compound together with the cells, tissues, non-human animals, or plants; and comparing to a control the RNA function suppression activity of the RNA of a gene having a sequence complementary to the polynucleotide sequence of either of the component (I) or (III),
where, the component (III) comprises a continuous polynucleotide sequence of 15 to 30 that has a sequence complementary to the target gene,
the component (II) is a nucleotide sequence or non-nucleotide sequence with a base length of from 0 bases to 10 kilobases (where, 0 bases means a bond), and
the component (I) is a polynucleotide sequence comprising a sequence complementary to the component (III).

38. A polynucleotide sequence for a target gene according to any of claims 1 to 4 wherein the component (III) comprises any type of 1 to 5 ribonucleotides continuing at the 18 to 25 ribonucleotides complementary to the target gene, and the component (I) comprises 18 to 25 ribonucleotides complementary to the 18 to 25 nucleotides of the component (III).

39. A method for synthesizing nucleotides for target genes including the following steps:
(i) preparing a single strand nucleotide comprising component (I) and (II) such that several nucleotides of the 3' terminal of component (II) are complementary to several nucleotides of component (I) or (II);
(ii) synthesizing component (III) based on nucleotide synthesis enzyme activity using this single strand nucleotide comprising components (I) and (II), or introducing this single strand nucleotide comprising components (I) and (II) into a cell and synthesizing component (III) based on the nucleotide synthesis enzyme activity present inside the cell.

40. A nucleotide for a randomized target gene obtained by the method of claim 39, wherein the components (I) and (III) are random oligonucleotides.


SEQUENCE LISTING

<110> Otsuka Pharmaceutical Co., Ltd.

<120> Polynucleotides for a target gene

<130> 20045D

<150> JP 2002-46889
<151> 2002-02-22

<160> 21

<170> PatentIn version 3.1

<210> 1
<211> 52
<212> RNA
<213> Artificial Sequence

<220>
<223> uGL3.12RNA

<400> 1
cuuacgcuga guacuucgau uuguccguuc gucgaaguac ucagcguaag uu 52

<210> 2
<211> 47
<212> RNA
<213> Artificial Sequence

<220>
<223> uGL3.7RNA

<400> 2

cuuacgcuga guacuucgau uuguccucga aguacucagc guaaguu 47

<210> 3
<211> 12
<212> RNA
<213> Artificial Sequence

<220>
<223> component sequence of uGL3.12RNA

<400> 3
uuuguccguu cg 12

<210> 4
<211> 7
<212> RNA
<213> Artificial Sequence

<220>
<223> component sequence of uGL3.7RNA

<400> 4
uuugucc 7

<210> 5
<211> 21
<212> RNA
<213> Artificial Sequence

<220>
<223> forward sequence of EGFP 936-954

<400> 5

gacccgcgcc gaggugaagu u                                                21

<210>  6
<211>  21
<212>  RNA
<213>  Artificial Sequence

<220>
<223>  reverse sequence of EGFP 936-954

<400>  6
cuucaccucg gcgcgggucu u                                                21

<210>  7
<211>  36
<212>  DNA
<213>  Artificial Sequence

<220>
<223>  U6P.F

<400>  7
tctttggaat tcaaggtcgg gcaggaagag ggccta                                36

<210>  8
<211>  40
<212>  DNA
<213>  Artificial Sequence

<220>
<223>  U6CSP45R

<400>  8
tgctgccgaa gcgagcacgg tgtttcgaac tttccacaag                            40

<210> 9
<211> 61
<212> DNA
<213> Artificial Sequence

<220>
<223> U6P.R

<400> 9
aaaaattcta gatgtaaaaa tagtgttgtg tgcctaggat atgtgctgcc gaagcgagca 60
c 61

<210> 10
<211> 30
<212> DNA
<213> Artificial Sequence

<220>
<223> mH1.F

<400> 10
tttttgaatt catgcaaatt acgcgctgtg 30

<210> 11
<211> 54
<212> DNA
<213> Artificial Sequence

<220>
<223> mH1.R

<400> 11
ccaagggtcg acaaaaagat atctggatcc gtctagaccg gccgccacta taag 54

<210> 12
<211> 47
<212> DNA
<213> Artificial Sequence

<220>
<223> hLaminAC-4.H1F

<400> 12
aaaggtctag accctaaaga ataagtcttc tccagctcct taaggag 47

<210> 13
<211> 45
<212> DNA
<213> Artificial Sequence

<220>
<223> hLaminAC-4.R

<400> 13
attatgtcga caaaaagaat aagtcttctc cagctcctta aggag 45

<210> 14
<211> 32
<212> DNA
<213> Artificial Sequence

<220>
<223> Neo-Nde.F

<400> 14
agaattccat atgtggaatg tgtgtcagtt ag 32

<210> 15
<211> 32
<212> DNA
<213> Artificial Sequence

<220>
<223> Neo-Nde.R

<400> 15
agaattccat atgaggtcga cggtatacag ac 32

<210> 16
<211> 30
<212> DNA
<213> Artificial Sequence

<220>
<223> biH1pro.SacI

<400> 16
tttttgagct catgcaaatt acgcgctgtg 30

<210> 17
<211> 31
<212> DNA
<213> Artificial Sequence

<220>
<223> biH1pro.SpeI-2

<400> 17
tttttactag ttggtctaga ccggccgcca c 31

<210> 18
<211> 42
<212> DNA
<213> Artificial Sequence

<220>
<223> 25s#4F

<400> 18
cgaaaaaaag cataaggcta tgaagagata cgcctttttg gt 42

<210> 19
<211> 44
<212> DNA
<213> Artificial Sequence

<220>
<223> 25s#4R

<400> 19
ctagaccaaa aaggcgtatc tcttcatagc cttatgcttt tttt 44

<210> 20
<211> 52
<212> DNA
<213> Artificial Sequence

<220>
<223> ST-UHGL3#4F

<400> 20
tggaaagttc gaaaaaaagc ataaggctat gaagagatac gccttaaggc gt 52

<210> 21
<211> 53
<212> DNA
<213> Artificial Sequence

<220>
<223> ST-UHGL3#4R

<400> 21
cacgggtcta gaccaaaaag cataaggcta tgaagagata cgccttaagg cgt 53